# EUROPEAN PATENT SPECIFICATION

(11) **EP 1 708 619 B1**
(45) Date of publication and mention of the grant of the patent: **02.10.2019**
(21) Application number: 05722437.0
(22) Date of filing: 18.01.2005
(51) Int. Cl.: A61B 10/00

(54) **BIOPSY NEEDLE DEVICE**
BIOPSIE-NADELVORRICHTUNG
DISPOSITIF D'AIGUILLE DE BIOPSIE

(30) Priority: 28.01.2004 US 766369; 30.11.2004 US 631706 P
(43) Date of publication of application: 11.10.2006
(73) Proprietor: Specialized Health Products Inc., Bountiful, UT 84010-8321 (US)
(72) Inventor: HORNER, Shawn K., Woods Cross, UT 84087 (US); FERGUSON, F. Mark, Salt Lake City, UT 84124 (US); SOLOMON, Donald, D., North Salt Lake, UT 84054 (US)
(74) Representative: Alton, Andrew
(86) International application number: PCT/US2005/001345
(87) International publication number: WO 2005/072621

(56) References cited:
- WO-A1-02/24077
- WO-A1-99/52584
- US-A- 5 313 958
- US-A- 5 313 958
- US-A- 6 004 294
- US-A1- 2003 100 868
- US-A1- 2003 114 797
- US-A1- 2003 114 797

## Description

### BACKGROUND

### 1. Technical Field

The present disclosure generally relates to safety shields for medical needles, and more particularly, to safety shields that protect a needle point of a medical needle for tissue biopsy.

### 2. Description of the Related Art

Problems associated with inadvertent needle sticks are well known in the art of blood sampling, percutaneous medication injection and other medical procedures involving use of medical needles. Significant attention has been focused on needle stick problems due to the contemporary sensitivity of exposure to AIDS, Hepatitis and other serious blood-borne pathogen exposures.

Procedures for removing a needle from a patient commonly require a technician to use one hand to place pressure at the wound site where the needle is being withdrawn, while removing the needle device with the other hand. It is also common practice for an attending clinician to give higher priority to care for the patient than is given to disposal of a needle. In the case of typical needle devices without safety shields, such priority either requires the convenience of an available sharps container within reach or another means for safe disposal without leaving the patient's side. Providing adequate care while following safety procedures is often compounded by the patient's physical condition and mental state, such as in burn units and psychiatric wards. Under such conditions, it is difficult to properly dispose of a used needle while caring for a patient.

The widespread knowledge and history associated with needle care and disposal problems have resulted in numerous devices for preventing accidental needle sticks. Problems of current safety devices include difficulty of use and high cost due to their complexity and number of parts.

Other known devices employ sheaths that are telescoping, pivoting, etc. These devices, however, may be disadvantageously cumbersome to activate. Further drawbacks of current devices include high manufacturing cost due to complexity and the number of parts. Thus, these type prior art devices may not adequately and reliably shield medical needle apparatus to prevent hazardous exposure.

US2003/0100868 describes a medical needle shield apparatus that includes a needle shield that is extensible from a retracted position to an extended position to enclose the distal end of a needle. The needle shield includes a binding member with defined binding surfaces that form an aperture for slidable receiving the needle between a retracted position and an extended position. The binding member also includes a drag-inducing member configured for slidable engagement with the needle and to creat a drag force with the needle. This drag force causes rotation of the binding member relative to the longitudinal axis of the needle so that the binding surfaces engage the needle to prevent slidable movement of the needle when the shield is in the extended position. The shield can be used with a needle device and can be moved from a proximal position to an extended position to cover the distal end of the needle of the needle device after use.

WO 02/24077 describes a biopsy needle that includes a cannula and a stylet slidably received in the cannula. The stylet has a distal end with at least one tissue receiving receiving. First and second biasing mechanisms are configured to urge the stylet and cannula respectively from respective retracted positions to respective extended positions. First and second latch mechanisms are configured to releasably hold the stylet and the cannula, respectively, in their retracted positions. A trigger mechanism is configured to cause the first latch mechanism to release the stylet when in a first position, and when the trigger mechanism is moved from the second position to a third position, the trigger mechanism causes the second latch mechanism to release the cannula.

Consequently, there remains a need to provide a more satisfactory solution for needle safety devices by overcoming the disadvantages and drawbacks of the prior art. Therefore, it would be desirable to provide a more adequate and reliable medical needle shield apparatus that employs a safety shield slidably movable along a medical needle to prevent hazardous exposure to a needle tip. Such a needle shield apparatus should be easily and reliably movable to shield a needle tip of a needle canula.

### SUMMARY

Accordingly, the present disclosure addresses a need for a medical needle shield apparatus for a medical needle for tissue biopsy that effectively and inexpensively protects a tip of a medical needle for tissue biopsy after use. The present disclosure resolves related disadvantages and drawbacks experienced in the art. More specifically, the apparatus of this invention constitute an important advance in the art of safety needle devices.

The invention provides a medical needle shield apparatus in accordance with claim 1.

The binding member may include a substantially planar aperture plate that includes the binding surfaces that form the aperture. The at least one drag inducing member may include a pair of arms extending from the aperture plate.

The first housing may include a locking configuration that mates with a groove of the second housing to facilitate releasable engagement of the first housing and the second housing.

The actuating mechanism may include a slide mounted with the needle cannula. The slide facilitates axial movement of the needle cannula. The actuating mechanism may also include a biasing member that engages the slide to bias the needle cannula in a distal direction. The actuating mechanism may include a trigger that is connected to the biasing member for actuation thereof.

The second housing may include an inner housing that is disposed with the binding member. The inner housing may define at least one blocking member extending from an interior surface thereof. The at least one blocking member is engageable with the binding member for urging the binding member to a binding orientation.

The inner needle can include a lateral recess disposed adjacent a distal end thereof. Various methods of use of the medical needle shield apparatus are contemplated in accordance with the principles of the present disclosure.

### BRIEF DESCRIPTION OF THE DRAWINGS

The foregoing and other features and advantages of the present invention will be more fully understood from the following detailed description of the exemplary embodiments, taken in conjunction with the accompanying drawings in which :
FIGURE 1 is a perspective view of one particular embodiment of a medical needle shield apparatus in accordance with the principles of the present disclosure;
FIGURE 2 is a perspective view of the medical needle shield apparatus shown in FIGURE 1 with parts separated;
FIGURE 3 is a cutaway perspective view of the medical needle shield apparatus shown in FIGURE 1 with a housing section removed;
FIGURE 4 is a perspective view of the indicated area of detail shown in FIGURE 3;
FIGURE 5 is a cutaway cross-sectional view of the medical needle shield apparatus shown in FIGURE 1;
FIGURE 6 is a cross-sectional view of the indicated area of detail shown in FIGURE 5;
FIGURE 7 is an enlarged perspective view of a binding member of the medical needle shield apparatus shown in FIGURE 1;
FIGURE 8 is a cutaway perspective view of the medical needle shield apparatus shown in FIGURE 1 with a housing section removed;
FIGURE 9 is a perspective view of the indicated area of detail shown in FIGURE 8;
FIGURE 10 is a cutaway cross-sectional view of the medical needle shield apparatus shown in FIGURE 1;
FIGURE 11 is a cross-sectional view of the indicated area of detail shown in FIGURE 10;
FIGURE 12 is a cross-sectional view of the indicated area of detail shown in FIGURE 10;
FIGURE 13 is a cross-sectional view of the indicated area of detail shown in FIGURE 10;
FIGURE 14 is a cutaway perspective view of the medical needle shield apparatus shown in FIGURE 1 with a housing section removed;
FIGURE 15 is a perspective view of the indicated area of detail shown in FIGURE 14;
FIGURE 16 is a cutaway cross-sectional view of the medical needle shield apparatus shown in FIGURE 1;
FIGURE 17 is a cross-sectional view of the indicated area of detail shown in FIGURE 16;
FIGURE 18 is a cross-sectional view of the indicated area of detail shown in FIGURE 16;
FIGURE 19 is a cutaway perspective view of the medical needle shield apparatus shown in FIGURE 1 with a housing section removed;
FIGURE 20 is a perspective view of the indicated area of detail shown in FIGURE 19;
FIGURE 21 is a cutaway cross-sectional view of the medical needle shield apparatus shown in FIGURE 1;
FIGURE 22 is a cross-sectional view of the indicated area of detail shown in FIGURE 21;
FIGURE 23 is a cross-sectional view of the indicated area of detail shown in FIGURE 21;
FIGURE 24 is a cutaway perspective view of the medical needle shield apparatus shown in FIGURE 1 with parts separated;
FIGURE 25 is a cutaway cross-sectional view of the medical needle shield apparatus shown in FIGURE 1 with parts separated;
FIGURE 26 is a cross-sectional view of the indicated area of detail shown in FIGURE 25;
FIGURE 27 is a cutaway top view of the medical needle shield apparatus shown in FIGURE 1;
FIGURE 28 is a cutaway top view of the medical needle shield apparatus shown in FIGURE 1 with parts separated;
FIGURE 29 is a cutaway cross-sectional top view of the medical needle shield apparatus shown in FIGURE 1;
FIGURE 30 is a cross-sectional view of the indicated area of detail shown in FIGURE 29;
FIGURE 31 is a cutaway cross-sectional top view of the medical needle shield apparatus shown in FIGURE 1 with parts separated;
FIGURE 32 is a cross-sectional view of the indicated area of detail shown in FIGURE 31;
FIGURE 33 is a perspective view of the medical needle shield apparatus shown in FIGURE 1 with parts separated;
FIGURE 34 is a perspective view of another embodiment of a medical needle shield apparatus in accordance with the principles of the present disclosure;
FIGURE 35 is a perspective view of the medical needle shield apparatus shown in FIGURE 34 with parts separated;
FIGURE 36 is a cutaway perspective view of the medical needle shield apparatus shown in FIGURE 34 with a housing section removed;
FIGURE 37 is a cutaway cross-sectional view of the medical needle shield apparatus shown in FIGURE 34;
FIGURE 38 is a cross-sectional view of the indicated area of detail shown in FIGURE 37;
FIGURE 39 is a cutaway perspective view of the medical needle shield apparatus shown in FIGURE 34 with a housing section removed;
FIGURE 40 is a perspective view of the indicated area of detail shown in FIGURE 39;
FIGURE 41 is a cutaway cross-sectional view of the medical needle shield apparatus shown in FIGURE 34;
FIGURE 42 is a cross-sectional view of the indicated area of detail shown in FIGURE 41;
FIGURE 43 is a cross-sectional view of the indicated area of detail shown in FIGURE 41;
FIGURE 44 is a cross-sectional view of the indicated area of detail shown in FIGURE 41;
FIGURE 45 is a cross-sectional view of the indicated area of detail shown in FIGURE 41;
FIGURE 46 is a cutaway perspective view of the medical needle shield apparatus shown in FIGURE 34 with a housing section removed;
FIGURE 47 is a perspective view of the indicated area of detail shown in FIGURE 34;
FIGURE 48 is a cutaway cross-sectional view of the medical needle shield apparatus shown in FIGURE 34;
FIGURE 49 is a cross-sectional view of the indicated area of detail shown in FIGURE 48;
FIGURE 50 is a cross-sectional view of the indicated area of detail shown in FIGURE 48;
FIGURE 51 is a cutaway perspective view of the medical needle shield apparatus shown in FIGURE 34 with a housing section removed;
FIGURE 52 is a perspective view of the indicated area of detail shown in FIGURE 51;
FIGURE 53 is a cutaway cross-sectional view of the medical needle shield apparatus shown in FIGURE 34;
FIGURE 54 is a cross-sectional view of the indicated area of detail shown in FIGURE 53;
FIGURE 55 is a cutaway cross-sectional view of the medical needle shield apparatus shown in FIGURE 34;
FIGURE 56 is a cross-sectional view of the indicated area of detail shown in FIGURE 55;
FIGURE 57 is a cutaway cross-sectional view of the medical needle shield apparatus shown in FIGURE 34;
FIGURE 58 is a cross-sectional view of the indicated area of detail shown in FIGURE 57;
FIGURE 59 is a cutaway perspective view of the medical needle shield apparatus shown in FIGURE 34 with parts separated;
FIGURE 60 is a cutaway cross-sectional view of the medical needle shield apparatus shown in FIGURE 34 with parts separated;
FIGURE 61 is a cross-sectional view of the indicated area of detail shown in FIGURE 60;
FIGURE 62 is a perspective view of another embodiment of a medical needle shield apparatus in accordance with the principles of the present disclosure;
FIGURE 63 is perspective view of the apparatus shown in FIGURE 62 in an alternate position;
FIGURE 64 is a perspective view of the apparatus shown in FIGURE 62 in an alternate position;
FIGURE 65 is a perspective view of another embodiment of a medical needle shield apparatus in accordance with the principles of the present disclosure;
FIGURE 66 is a perspective view of the medical needle shield apparatus shown in FIGURE 65 with parts separated;
FIGURE 67 is a cutaway perspective view of the medical needle shield apparatus shown in FIGURE 65 with a housing section removed;
FIGURE 68 is a cutaway cross-sectional view of the medical needle shield apparatus shown in FIGURE 65;
FIGURE 69 is a cutaway perspective view of the medical needle shield apparatus shown in FIGURE 65 with a housing section removed;
FIGURE 70 is a cutaway cross-sectional view of the medical needle shield apparatus shown in FIGURE 65;
FIGURE 71 is a cross-sectional view of the medical needle shield apparatus shown in FIGURE 65;
FIGURE 72 is a cutaway perspective view of the medical needle shield apparatus shown in FIGURE 65 with a housing section removed;
FIGURE 73 is a cross-sectional view of the medical needle shield apparatus shown in FIGURE 65;
FIGURE 74 is a cutaway perspective view of the medical needle shield apparatus shown in FIGURE 65 with a housing section removed;
FIGURE 75 is a cross-sectional view of the medical needle shield apparatus shown in FIGURE 65;
FIGURE 76 is a cutaway perspective view of the medical needle shield apparatus shown in FIGURE 65 with a housing section removed;
FIGURE 77 is a cross-sectional view of the medical needle shield apparatus shown in FIGURE 65;
FIGURE 78 is a cutaway perspective view of the medical needle shield apparatus shown in FIGURE 65 with a housing section removed;
FIGURE 79 is a cutaway cross-sectional view of the medical needle shield apparatus shown in FIGURE 65 with parts separated;
FIGURE 80 is a cutaway cross-sectional view of the medical needle shield apparatus shown in FIGURE 65 with parts separated; and
FIGURE 81 is a cross-sectional view of the medical needle shield apparatus shown in FIGURE 65.

### DETAILED DESCRIPTION OF THE EXEMPLARY EMBODIMENTS

The exemplary embodiments of the medical needle shield apparatus and methods of operation disclosed are discussed in terms of medical needles for tissue biopsy, and more particularly, in terms of needle shield apparatus employed with a needle cannula that prevent hazardous exposure to a needle tip, including, for example, inadvertent needle sticks. It is envisioned that the present disclosure, however, finds application to a wide variety of cannula needles, stylettes and devices for tissue biopsy, sampling, etc. Such devices may include mechanisms for actuating a needle cannula, such as, for example, semi-automatic soft tissue biopsy needles, semi-automatic soft tissue biopsy guns, automatic soft tissue biopsy devices, etc.

In the discussion that follows, the term "proximal" refers to a portion of a structure that is closer to a clinician, and the term "distal" refers to a portion that is further from the clinician. As used herein, the term "subject" refers to a patient having tissue biopsy using the medical needle shield apparatus. According to the present disclosure, the term "clinician" refers to an individual performing tissue collection, installing or removing a needle cannula from a medical needle shield apparatus and may include support personnel.

The following discussion includes a description of the medical needle shield apparatus, followed by a description of the method of operating the medical needle shield apparatus in accordance with the present disclosure. Reference will now be made in detail to the exemplary embodiments of the disclosure, which are illustrated in the accompanying figures.

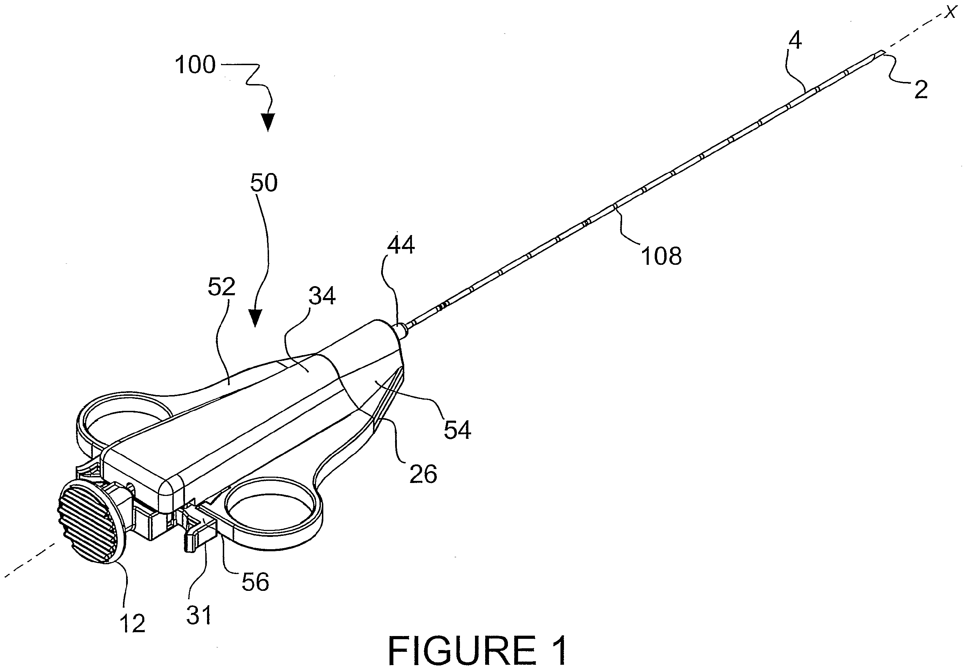

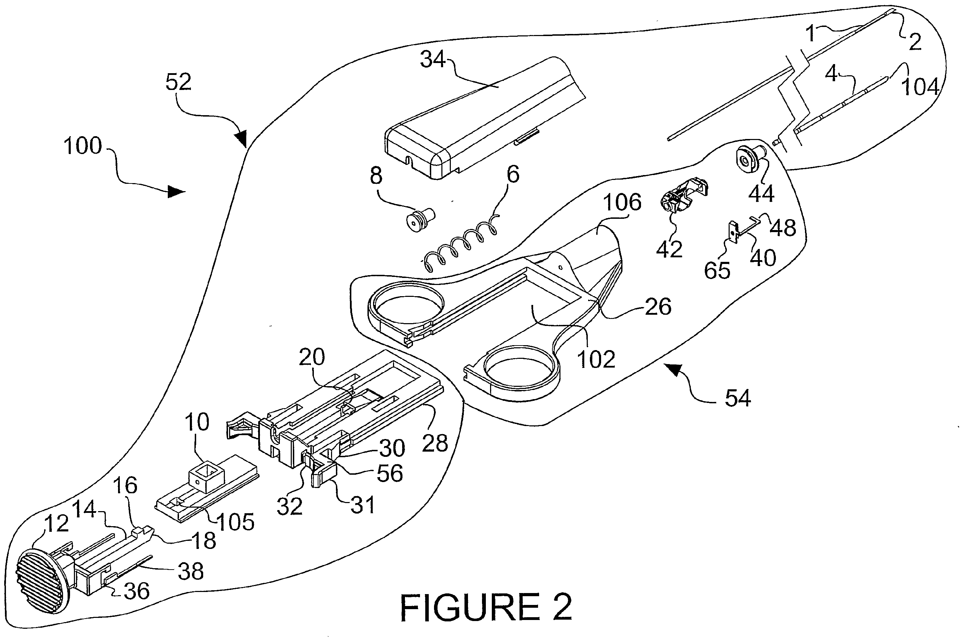

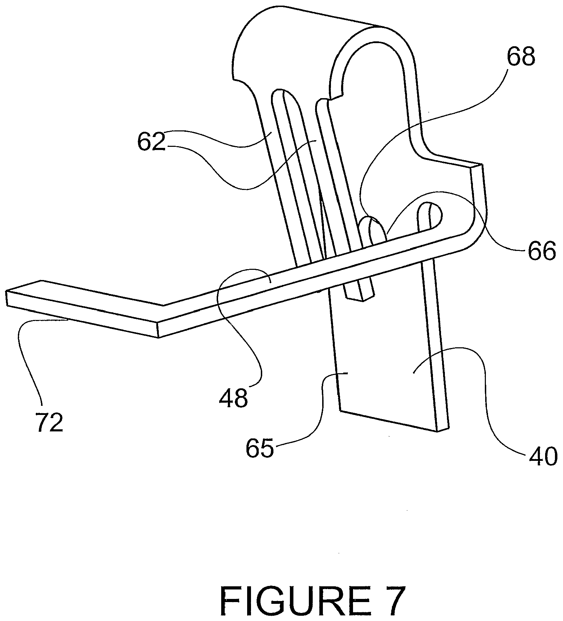

Turning now to the figures, wherein like components are designated by like reference numerals throughout the several views. Referring initially to FIGURES 1-7, there is illustrated a medical needle shield apparatus 100, constructed in accordance with the principals of the present disclosure. Medical needle shield apparatus 100 has a body 50 that includes a first housing, such as, for example, body core 52. Body core 52 includes an actuating mechanism that actuates a needle cannula 4 disposed therewith. The actuating mechanism, an example of which is described below, advances needle cannula 4 to facilitate tissue sampling, as will be discussed.

Needle cannula 4 includes a cutting edge, echogenic features and depth markings 108. An inner needle, such as, for example, stylette 2 is disposed for slidable movement within needle cannula 4. Needle cannula 4 is concentric with stylette 2. Stylette 2 has a cutting edge and a recess 1 configured to capture tissue samples, as will be discussed.

Body core 52 encloses components disposed therein with a core cover 34. It is envisioned that core cover 34 may be variously configured and dimensioned such as, for example, rectangular, spherical, etc. It is further envisioned that core cover 34 may be assembled by any appropriate process such as, for example, snap fit, adhesive, solvent weld, thermal weld, ultrasonic weld, screw, rivet, etc. Alternatively, body core 52 may be monolithically formed or integrally assembled of multiple housing sections and may be substantially transparent, opaque, etc. Body core 52 may include ribs, ridges, etc. to facilitate manipulation of medical needle shield apparatus 100.

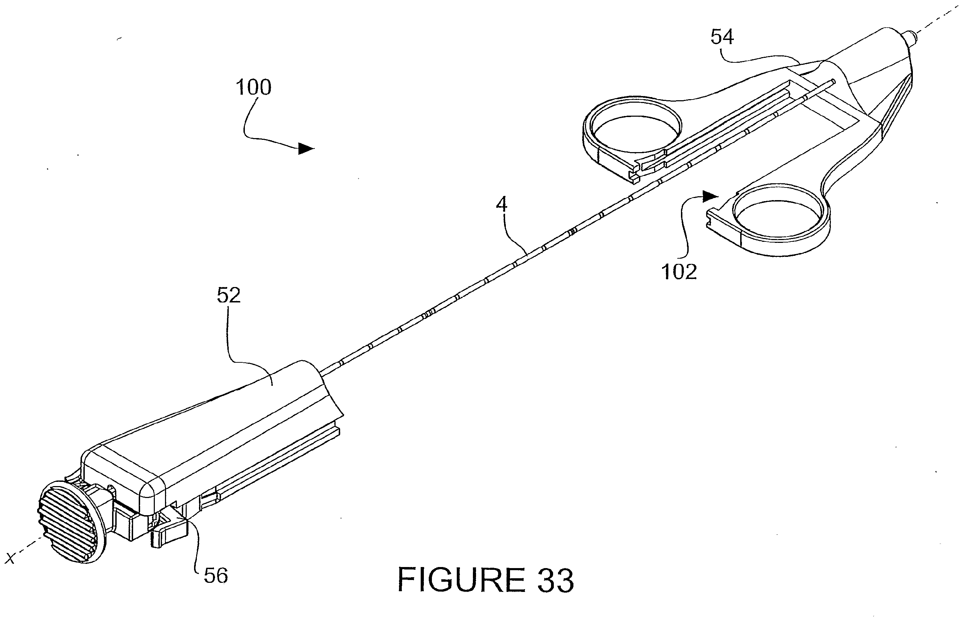

A second housing, such as, for example, a body base 54 includes a base handle 26. Base handle 26 defines a cavity 102 configured for receipt of body core 52 such that body core 52 is releasably engageable with body base 54. Needle cannula 4 is disposed for slidable movement with body base 54 such that body base 54 is extensible from a retracted position (FIG. 1) to an extended position (FIG. 33) to enclose a sharp distal end 104 of needle cannula 4.

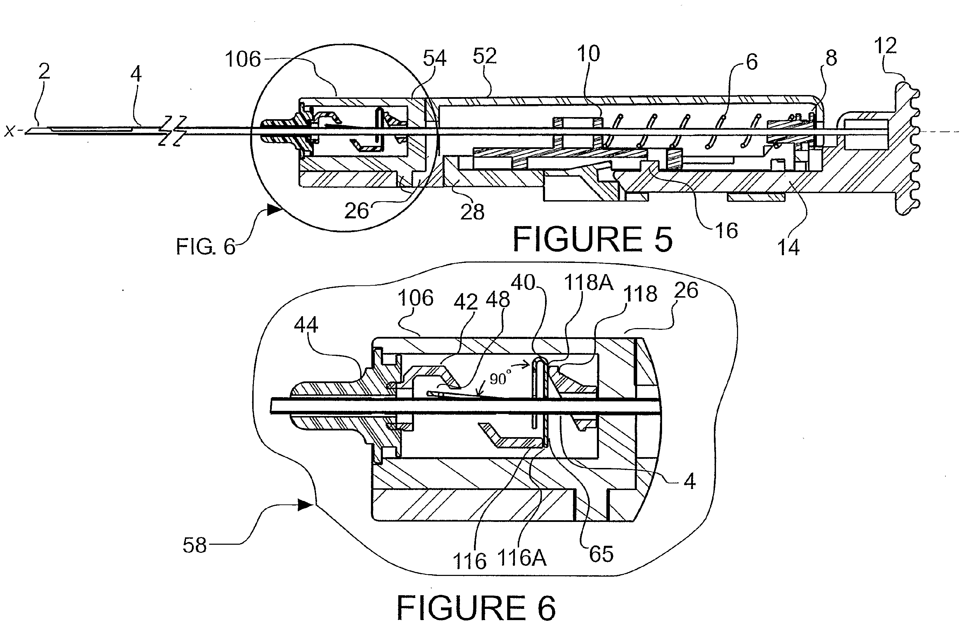

Body base 54 includes a binding member 40 that defines binding surfaces 68 (FIGS. 6-7). Binding surfaces 68 form an aperture 66 configured for slidable receipt of needle cannula 4 between the retracted position and the extended position. Aperture 66 is formed in an aperture plate 65. Binding member 40 may be monolithically formed and aperture plate 65 has a rectangular, generally planar configuration with sufficient stiffness to produce forces for binding needle cannula 4, as will be discussed. It is envisioned that aperture plate 65 may have an arcuate surface, undulating, etc. It is further envisioned that aperture plate 65 may have various degrees of stiffness according to the requirements of a particular application.

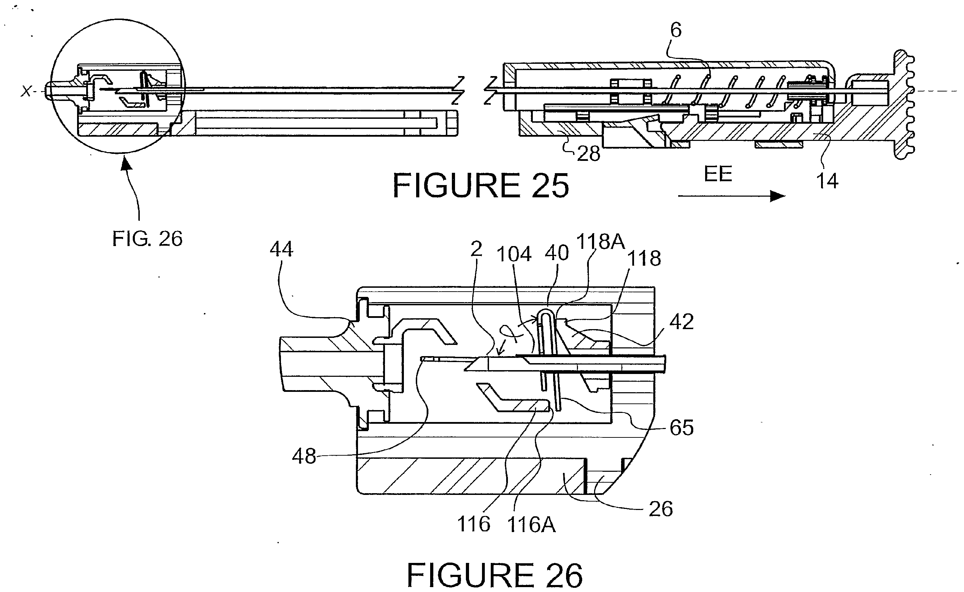

Binding member 40 includes at least one drag inducing member, such as, for example, friction members 62. Friction members 62 engage needle cannula 4 during slidable receipt of needle cannula 4 to create a drag force with needle cannula 4 between the retracted position and the extended position. Inner housing 42 includes blocking members 116, 118 configured to engage binding member 40 and are disposed not to interfere with needle cannula 4. Blocking members 116, 118 define surfaces 116A, 118A respectively, that facilitate disposal of aperture plate 65 from a non-binding or sliding orientation (FIGURE 6) to a binding orientation (FIGURE 26). Blocking members 116 and/or 118 cause binding member 40 to move to the binding orientation, in conjunction with the frictional drag force created between friction members 62 and needle cannula 4, as body base 54 is manipulated in the distal direction along longitudinal axis *x.* The force created by blocking members 116 and/or 118 acts in a direction opposite to the drag force, causing a force couple, which rotates binding member 40 to the binding orientation.

The frictional drag force in conjunction with blocking members 116 and/or 118 generates a canting force and inclination of aperture plate 65, relative to longitudinal axis *x.* The canting force urges rotation of binding member 40 and causes a lever or moment of end sensing member 48, which is opposed by needle cannula 4 to prevent rotation of binding member 40 in the sliding orientation.

Frictional members 62 may be monolithically formed with binding member 40 and extend from aperture plate 65 for alignment with aperture 66 and engagement with needle cannula 4. Frictional members 62 are spaced apart to facilitate sliding engagement with needle cannula 4. Such engagement creates the frictional drag force with needle cannula 4. It is envisioned that one or a plurality of friction members 62 may be employed. It is contemplated that frictional members 62 may be flexible or have flexible portions, which may be of varying flexibility according to the particular requirements of a needle application.

Binding member 40 also includes an end sensing member 48 extending therefrom. End sensing member 48 has a retainer 72 that is engageable with needle cannula 4 to prevent rotation of binding member 40. It is envisioned that retainer 72 may include ribs, projections, cavities, etc. for engagement with needle cannula 4 or that a portion of retainer 72 engages needle cannula 4.

Body base 54 includes a housing section 106 that encloses binding member 40 and adjacent components. It is envisioned that housing section 106 may be variously configured and dimensioned such as, for example, rectangular, spherical, etc. It is further envisioned that housing section 106 may be assembled by any appropriate process such as, for example, snap fit, adhesive, solvent weld, thermal weld, ultrasonic weld, screw, rivet, etc. Alternatively, body base 54 may be monolithically formed or integrally assembled of multiple housing sections and may be substantially transparent, opaque, etc. Body base 54 may include ribs, ridges, etc. to facilitate manipulation of medical needle shield apparatus 100.

The components of medical needle shield apparatus 100 can be fabricated from a material suitable for medical applications, such as, for example, polymerics or metals, such as stainless steel, depending on the particular medical application and/or preference of a clinician. Semi-rigid and rigid polymerics are contemplated for fabrication, as well as resilient materials, such as molded medical grade polypropylene. However, one skilled in the art will realize that other materials and fabrication methods suitable for assembly and manufacture, in accordance with the present disclosure, also would be appropriate.

The actuating mechanism includes a coring cannula slide 10 disposed within body core 52 and slidable relative thereto. Needle cannula 4 is mounted with coring cannula slide 10 for corresponding movement therewith. Coring cannula slide 10 is supported by a base safety slide 28 and enclosed by core cover 34. It is envisioned that coring cannula slide 10 may be variously configured and dimensioned such as, for example, rectangular, spherical, etc.

Base safety slide 28 is slidably received by cavity 102 for assembly of body core 52 and body base 54. Stylette 2 is mounted with a trigger-setting button 12 to facilitate actuation thereof and supported for movement relative to body core 52 via a bushing 8. Bushing 8 facilitates alignment and relative slidable movement of stylette 2 with body core 52 during use. It is envisioned that stylette 2 may be supported for movement with body core 52 without bushing 8.

Trigger-setting button 12 is operably connected to coring cannula slide 10 to facilitate actuation of needle cannula 4. Trigger-setting button 12 connects to coring cannula slide 10 via a biasing member, such as, for example, spring 6 and safety slide 28. Spring 6 is disposed within body core 52, concentrically about stylette 2. Spring 6 is mounted onto bushing 8 and into engagement with a proximal portion of coring cannula slide 10 for driving slide 10 during expansion. Trigger-setting button 12 is operative with a trigger-setting arm 14, which is configured to facilitate actuation of needle cannula 4 and stylette 2, as will be discussed.

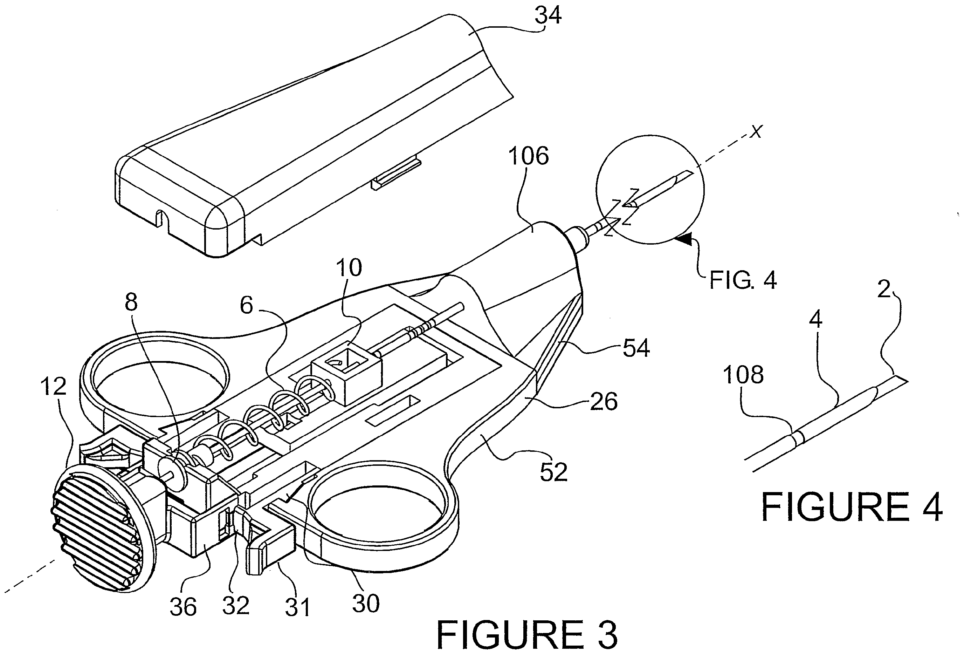

In a primary state of medical needle shield apparatus 100, as shown in FIGURES 1, 3 and 5, spring 6 is expanded and trigger-setting button 12 and coring cannula slide 10 are in a distal position on safety slide 28. Needle cannula 4 and stylette 2 are attached to coring cannula slide 10 and trigger-setting button 12, respectively, each in a distal position. In such a distal position, recess 1 of stylette 2 is enclosed by needle cannula 4, as shown in FIGURE 4. Binding member 40 is in a non-binding or sliding orientation, as will be discussed, which allows needle cannula 4 to slide freely.

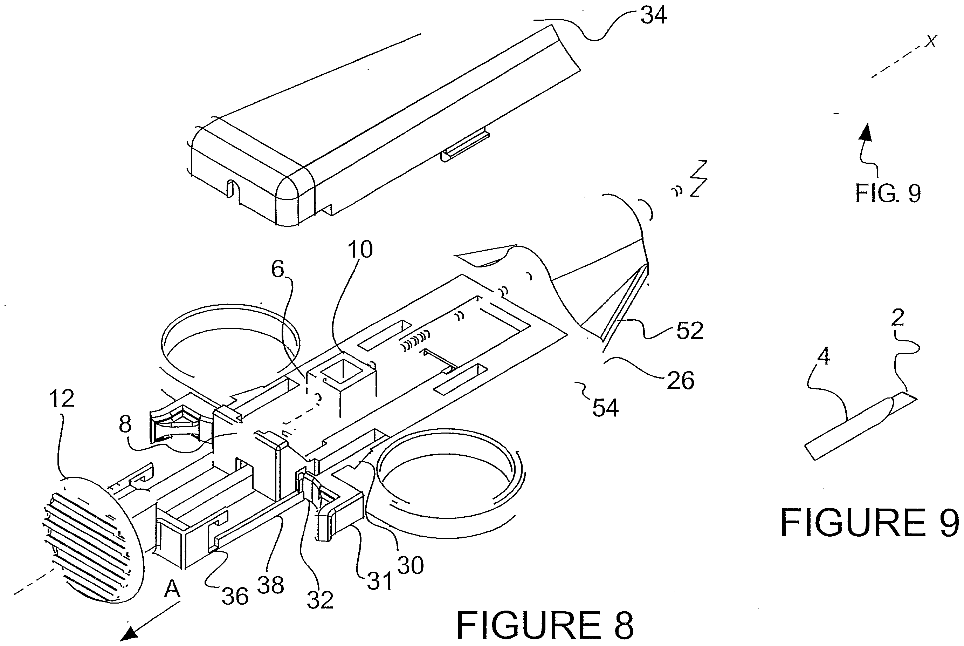

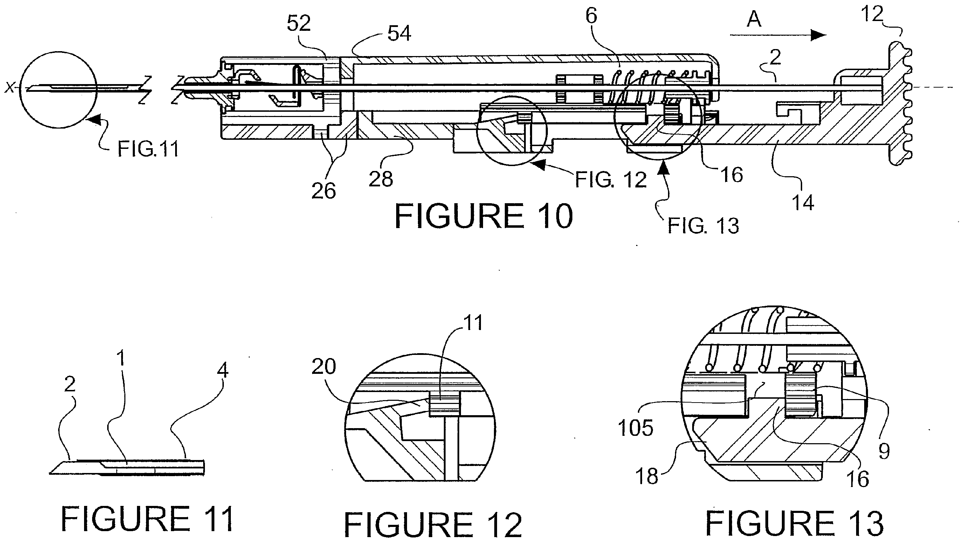

To set the actuating mechanism, trigger-setting button 12 is manipulated in a proximal direction along longitudinal axis x, in the direction of arrow A shown in FIGURES 8 and 10. Needle cannula 4 maintains enclosure of recess 1 of stylette 2, as shown in FIGURE 9. Trigger-setting arm 14 includes a trigger-setting tooth 16, which is configured for disposal within a groove 105 of coring cannula slide 10. Disposal of trigger-setting tooth 16 within groove 105 causes corresponding engagement with proximal slide stop 9, as shown in FIGURE 13. Correspondingly, a distal slide stop 11 of coring cannula slide 10 engages a compression lock 20, as shown in FIGURE 12. Proximal slide stop 9 and distal slide stop 11 thereby facilitate maintenance of the actuating mechanism in a set position. Proximal movement of trigger-setting button 12 also compresses spring 6 into a set position. In the set position, needle cannula 4 maintains enclosure of recess 1 of stylette 2, as shown in FIGURE 11.

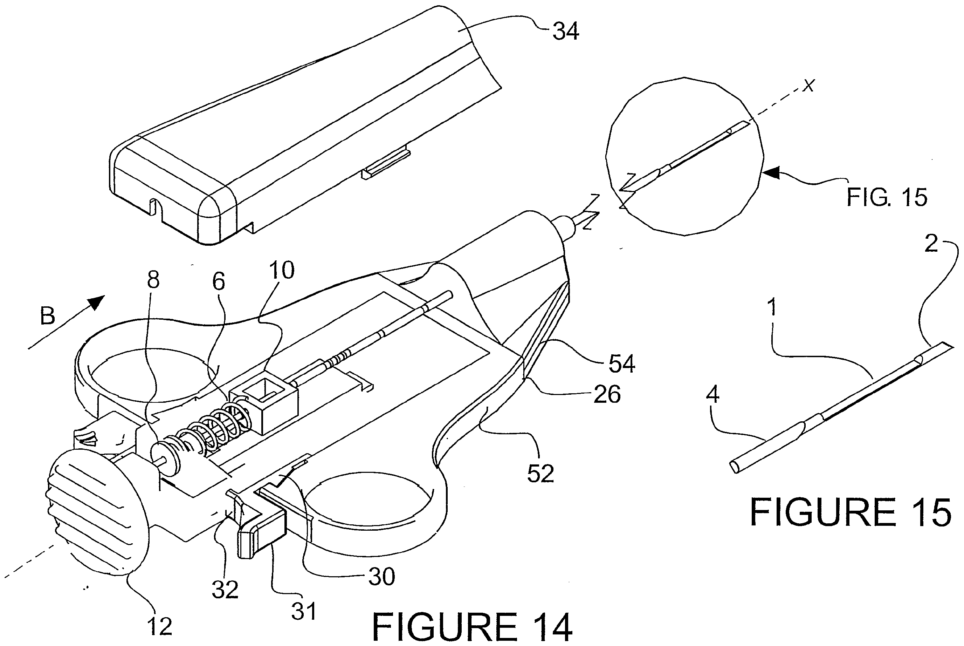

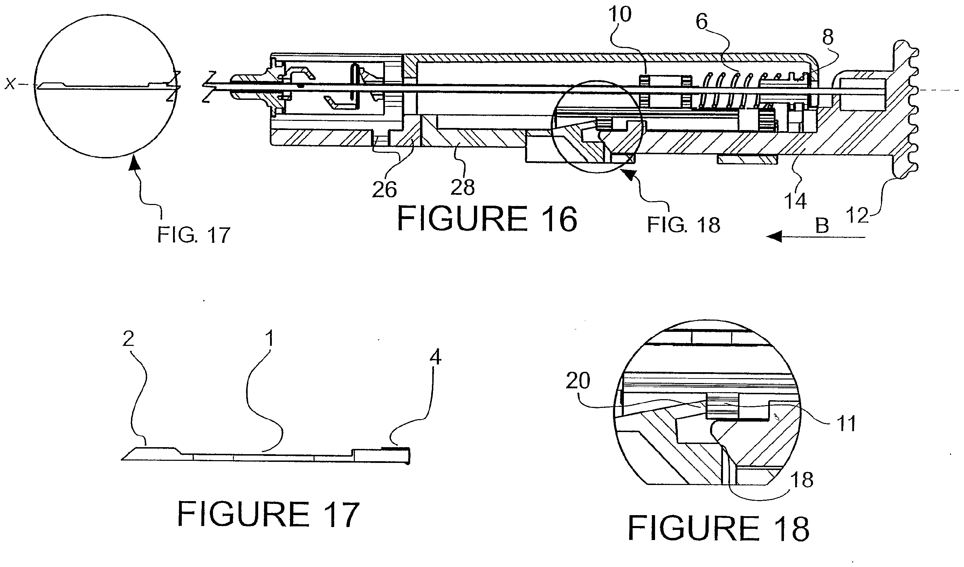

In contemplation of actuation of needle cannula 4, trigger-setting button 12 is manipulated in a distal direction, as shown by arrow B in FIGURES 14 and 16, until resistance is encountered. For example, a source of resistance to the distal manipulation can include engagement of a trigger core activating ramp 18 with compression lock 20, as shown in FIGURE 18. The distal manipulation of trigger-setting button 12 forces stylette 1 forward and exposes recess 1, as shown in FIGURES 15 and 17, while spring 6 is maintained in compression.

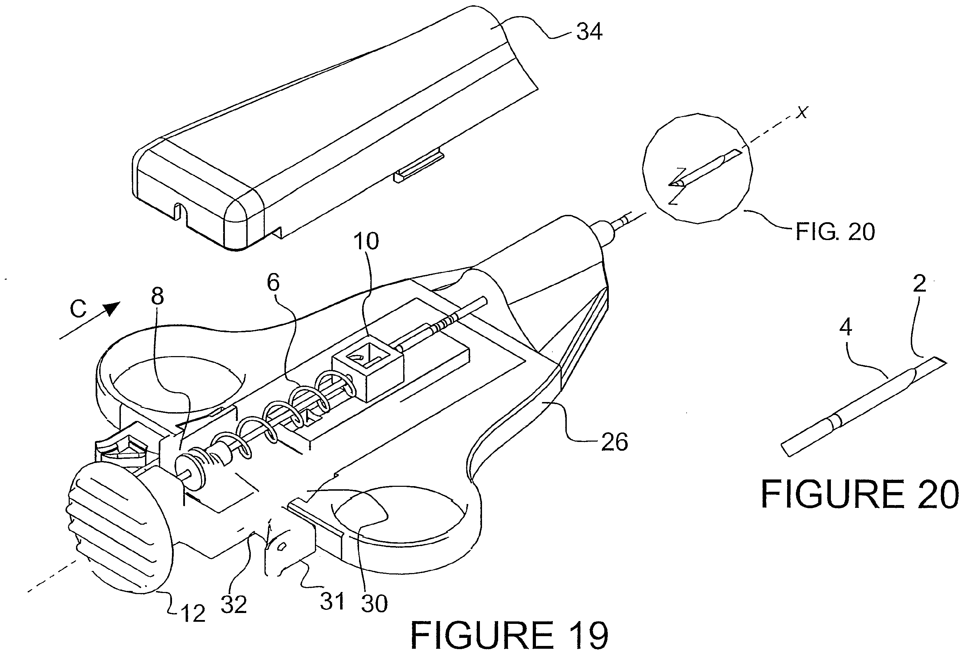

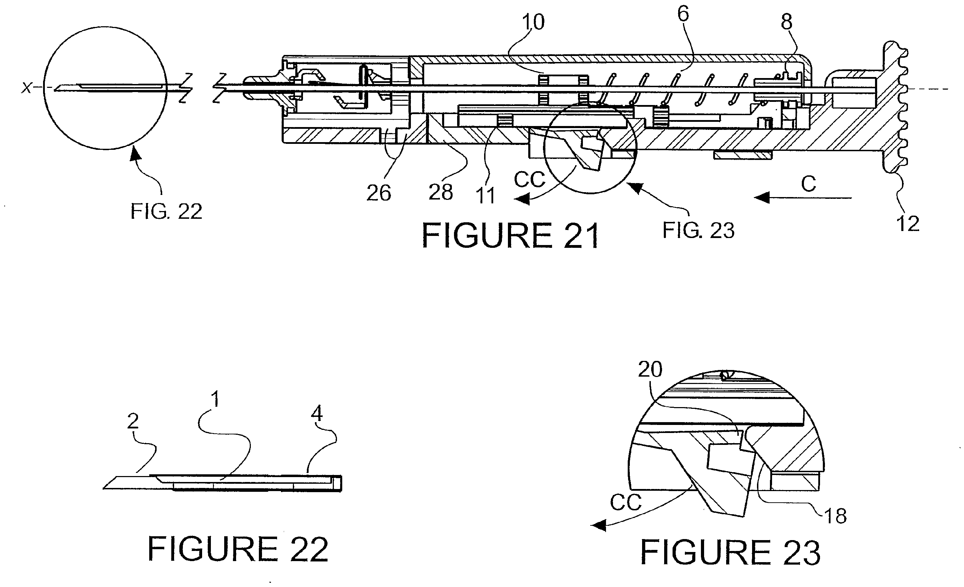

In an exemplary actuation, needle cannula 4 and stylette 2 are placed through soft tissue of a subject (not shown) to a desired location, which may be viewed via echogenic and depth markings 108. At the desired location, a tissue sample for biopsy enters recess 1. Trigger-setting button 12 is manipulated distally beyond the initial resistance of trigger core activating ramp 18 with compression lock 20, as shown by arrow C in FIGURE 21. Further manipulation of button 12 drives trigger core activating ramp 18 to deflect compression lock 20 such that distal slide stop 11 disengages from compression lock 20, as shown by arrow CC in FIGURES 21 and 23. Thus, coring cannula slide 10 is thrust, via spring 6, to a distal position, as shown by arrow D in FIGURE 19. Correspondingly, needle cannula 4 is thrust distally to core and enclose the soft tissue within recess 1, as shown in FIGURES 20 and 22. The captured soft tissue sample is then withdrawn from the subject.

The captured soft tissue sample is removed by resetting the actuating mechanism and distally manipulating button 12 to expose recess 1, as described above, outside of the subject. Additional soft tissue sampling may be repeated with medical needle shield apparatus 100.

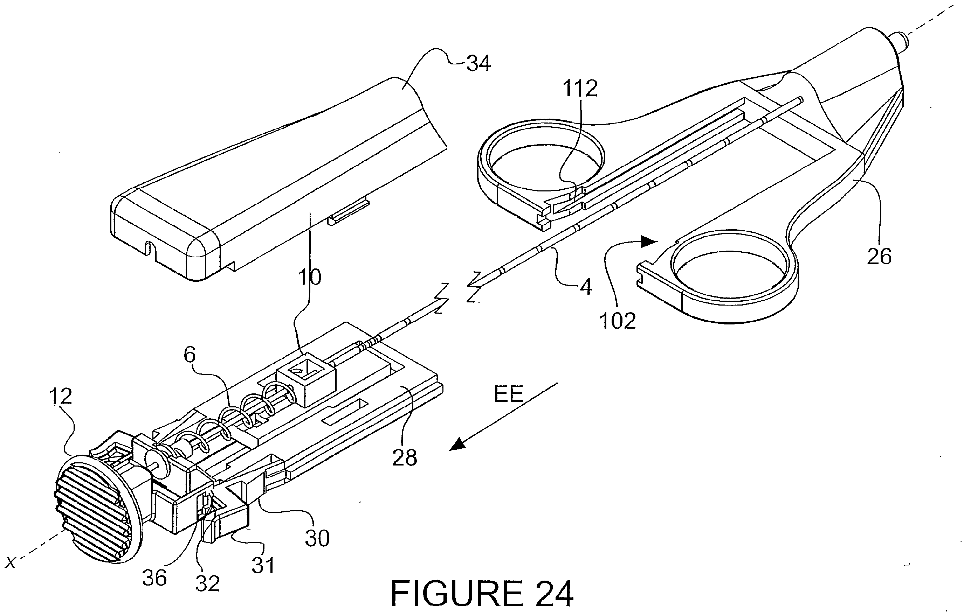

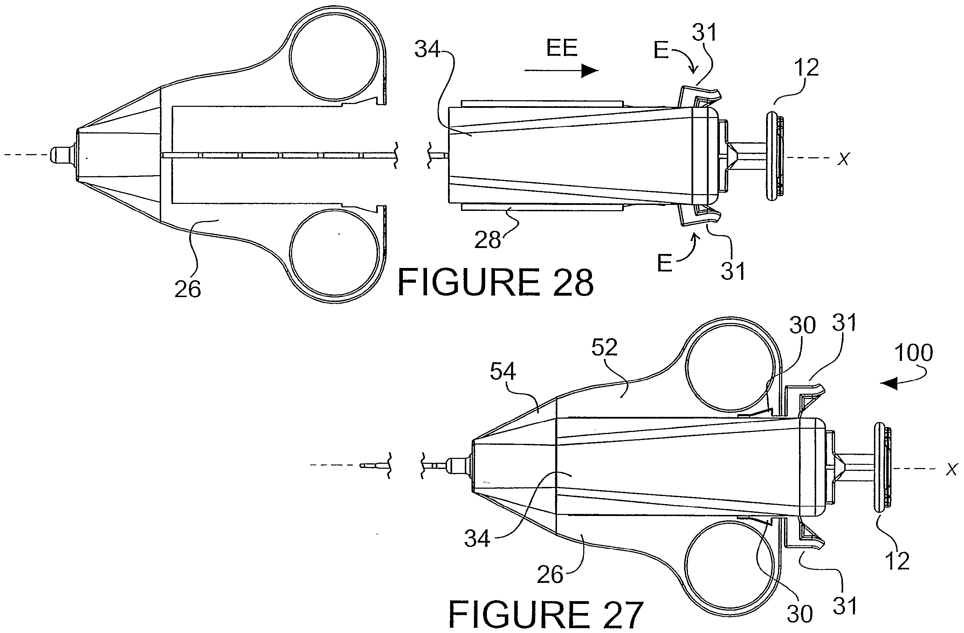

Referring to FIGURES 24-28, upon completion of soft tissue sampling, body core 52 and body base 54 may be disassembled via inward manipulation of base assembly locking arms 31, as shown by arrow E in FIGURE 28, and pulled proximally, in the direction shown by arrow EE, while base handle 26 is held in a fixed position. A base assembly locking tooth 30, disposed with safety slide 28 disengages from a mating groove 112 of base handle 26. This configuration advantageously separates body core 52 and body base 54.

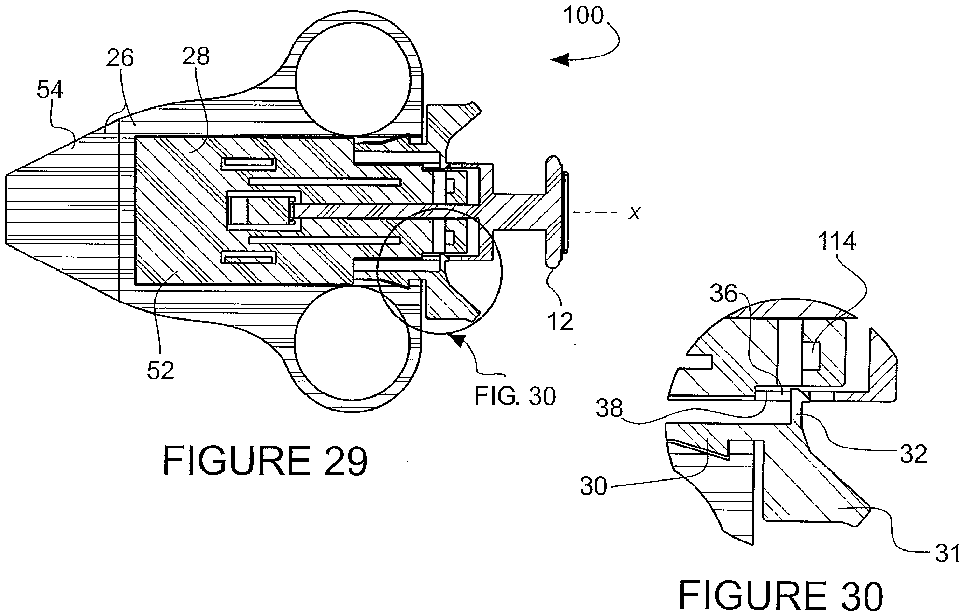

In an alternate embodiment, as shown in FIGURES 29-32, separating body core 52 and body base 54, in conjunction with manipulating base assembly locking arms 31 occurs only when medical needle shield apparatus 100 is in the primary state, as described. A trigger slide 38 of safety slide 28 provides a physical stop verification when the actuating mechanism is triggered. Trigger slide 38, in the triggered position, is aligned with base assembly locking arms 31 and prevents manipulation of arms 31.

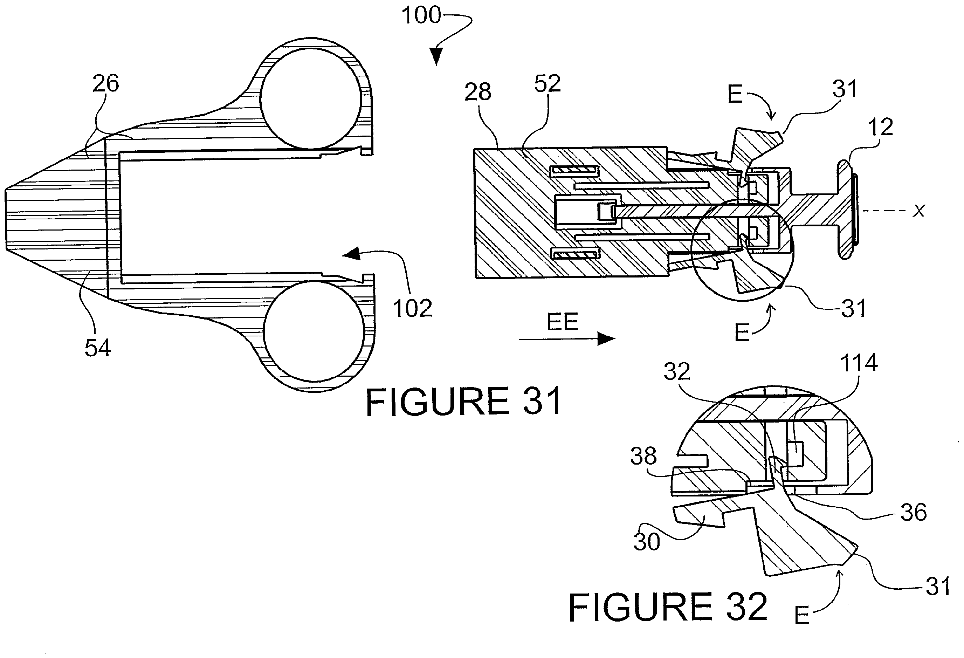

In the primary state, trigger slide 38 is disposed such that base assembly locking arms 31 are aligned with a trigger safety lockout gap 36, as shown in FIGURE 30. Base assembly locking arms 31 may be manipulated for inward movement. A base trigger safety lockout tooth 32 of arms 31 is deflected through the trigger safety lockout gap 36 and locks with an inner gap 114 of safety slide 28, as shown by arrow E in FIGURE 32. This configuration facilitates separation of body 50 and prevents activation of the actuating mechanism while binding member 40 is in a binding orientation, as will be discussed.

Body base 54 is extensible from a retracted position (FIGURE 1) to an extended position (FIGURE 33) to enclose distal end 104 of needle cannula 4. Binding member 40 is supported within body base 54 via an inner housing 42 and base handle 26, as shown in FIGURE 6. A cap 44 supports inner housing 42 with base handle 26 and facilitates slidable movement of needle cannula 4. Inner housing 42 is substantially cylindrical and may alternatively be variously configured and dimensioned such as, for example, rectangular, spherical, etc. It is contemplated that binding member 40 is rotatable about needle cannula 4 with inner housing 42. It is further contemplated that inner housing 42 is rotatable about needle cannula 4 with body base 54, or alternatively, relative to body base 54.

End sensing member 48 extends distally from aperture plate 65, parallel to needle cannula 4. End sensing member 48 is perpendicularly oriented relative to a plane defined by aperture plate 65. This perpendicular orientation facilitates inclination of aperture plate 40 for disposal in a binding or sliding orientation of binding member 40. It is envisioned that end sensing member 48 may be variously oriented with aperture plate 65 and may flexibly extend therefrom.

As needle cannula 4 is released from engagement with retainer 72, binding member 40 and end sensing member 48 rotate to the binding orientation. Corresponding rotation of aperture plate 65 causes binding surfaces 68 to frictionally engage needle cannula 4 and prevent movement thereof. Blocking members 116, 118 cause aperture plate 65 to move to the binding orientation as forces are imposed on body base 54 in either direction along longitudinal axis *x.*

In the binding orientation, proximal manipulation of needle cannula 4 causes aperture plate 65 to engage binding member 118 resulting in further counterclockwise inclination and enhanced frictional engagement of binding surfaces 68 with needle cannula 4. Distal manipulation of needle cannula 4 similarly causes aperture plate 65 to engage binding member 116 resulting in further counterclockwise inclination and enhanced frictional engagement of binding surfaces 68 with needle cannula 4. This configuration maintains needle cannula 4 within body base 54 to avoid hazardous exposure to distal end 104.

Aperture 66 is formed within aperture plate 65 for slidable engagement with needle cannula 4 during movement between the retracted position and the extended position of body base 54. Aperture 66 includes binding surfaces 68 formed on opposing sides of aperture 66 that engage needle cannula 4 to prevent movement thereof in the extended position of body base 54. It is contemplated that engagement to prevent movement of needle cannula 4 may include penetrating, frictional, interference, etc. It is envisioned that aperture 66 may have various geometric configurations, such as radial, polygonal, etc. It is further envisioned that aperture 66 may define an open cavity within aperture plate 65, such as, for example, "U" shaped and open to one or a plurality of edges of aperture plate 65.

The inclination of aperture plate 65 relative to longitudinal axis *x* facilitates sliding and binding, via binding surfaces 68, of needle cannula 4 within body base 54 to prevent hazardous exposure to distal end 104. For example, as shown in FIGURE 6, aperture plate 65 is oriented at an angle of approximately 90° relative to longitudinal axis *x* such that aperture plate 65 is disposed substantially perpendicular to needle cannula 4. In this sliding orientation, needle cannula 4 is free to slide within aperture 66. As needle cannula 4 is retracted and body base 54 is extended, needle cannula 4 continues to engage retainer 72 and aperture plate 65 maintains its perpendicular orientation relative to longitudinal axis *x.*

As body base 54 is manipulated to the extended position, friction members 62 in conjunction with blocking member 116, 118 cause aperture plate 65 to rotate counterclockwise relative to longitudinal axis *x.* As shown in FIGURE 26, aperture plate 65 rotates out of perpendicular alignment with needle cannula 4 such that aperture plate 65 is oriented at an angle *α*, which is less than 90° with respect to longitudinal axis *x.* It is contemplated that angle *α* may be measured from either side of aperture plate 65. Aperture plate 65 rotates to angle *α* and binding member 40 approaches the binding orientation.

For example, as shown in FIGURE 1, body base 54 is in the retracted position and needle cannula 4 is fully extended. Binding member 40 and aperture plate 65 are in a sliding orientation such that aperture plate 65 is substantially perpendicular to longitudinal axis *x.* Blocking members 116, 118 may engage aperture plate 65 to maintain aperture plate 65 in the perpendicular orientation. Blocking members 116, 118 may also maintain such orientation during extension of needle cannula 4 or may not engage needle cannula 4.

As needle cannula 4 is retracted and body base 54 is manipulated to the extended position (FIGURE 33), friction members 62 create a drag force via engagement with needle cannula 4 on binding member 40 and in conjunction with blocking member 118 cause aperture plate 65 to rotate in a counter-clockwise direction to the binding orientation. Blocking member surface 118A engages aperture plate 65 to facilitate rotation thereof from the perpendicular position into the binding orientation such that binding surfaces 68 engage needle cannula 4 to prevent axial movement thereof relative to body base 54 in the extended position. This configuration advantageously prevents hazardous exposure to needle cannula 4.

It is contemplated that binding surfaces 68 may include sharp edges to increase frictional engagement. It is further contemplated that the binding friction force may be created and varied by one or more altering factors, such as, for example, aperture 66 configuration and dimension, needle cannula 4 configuration and dimension, aperture plate 65 thickness, the dimension from blocking members 116, 118 contact point to the centerline of needle cannula 4 and the coefficient of friction between aperture 66 and needle cannula 4 depending on the particular requirements of a needle application. It is envisioned that friction members 62 may be configured to vary the drag force with variation of the inclination of aperture plate 65, which may be accomplished by geometric changes in the shape of friction members 62, such as wedge shapes or the inclusion of notches to engage needle cannula 4, or through the selective application of friction modifying materials or coatings such as oils, jells, greases, or coatings which increase friction. It is further envisioned that aperture 66 may be formed to accomplish the function of friction members 62.

In operation, medical needle shield apparatus 100, similar to that described in accordance with the principles of the present disclosure is provided for a tissue biopsy procedure. The components of medical needle shield apparatus 100 are fabricated, properly sterilized and otherwise prepared for storage, shipment and use. Referring to FIGURE 1, medical needle shield apparatus 100 is in the primary state such that needle cannula 4 is fully extended to enclose recess 1 of stylette 2 and the actuating mechanism is not in a triggered condition. Medical needle shield apparatus 100 is manipulated via handle 26.

Initially, the actuating mechanism is set by manipulating trigger-setting button 12 in a proximal direction to retract needle cannula 4 and stylette 2, and compress spring 6, as discussed. Body 50 is manipulated such that distal end 104 of needle cannula 4 is inserted into the tissue of a subject (not shown) for biopsy sampling. Proper positioning of distal end 104 in the tissue may be verified by echogenic features and depth markings 108. It is contemplated that positioning of distal end 104 or guidance thereof may be facilitated by imaging devices, such as, for example, radiological, ultrasound, etc.

Stylette 2 is advanced into the tissue to be sampled by manually urging trigger-setting button 12 in the distal direction. Advancement of stylette 2 exposes stylette recess 1. The tissue to be sampled then prolapses into stylette recess 1. Upon full depression of trigger-setting button 12, the actuating mechanism expands spring 6 and needle cannula 4 is released. Spring 6 drives needle cannula 4 distally. Distal end 104 of needle cannula 4 cuts and captures the tissue prolapsed into stylette recess 1.

Medical needle shield apparatus 100 is withdrawn from the subject. The actuating mechanism is set, as described, and stylette 2 is manually advanced exposing the tissue sample. The sample is caused to exit stylette recess 1 into a receptacle or the like. Tissue sampling employing medical needle shield apparatus 100 may be repeated as desired. Other methods of use are also contemplated.

During the tissue biopsy sampling procedure, body base 54 is in the retracted position and needle cannula 4 is extended, as shown in FIGURE 1. Binding member 40 is in a sliding orientation and needle cannula 4 may freely slide, as discussed. Upon completion of the tissue biopsy sampling procedure, distal end 104 is protected to prevent hazardous exposure thereto. Needle cannula 4 is retracted and body base 54 is manipulated to the extended position (FIGURE 33). Binding member 40 rotates to the binding orientation, as discussed, to prevent axial movement of needle cannula 4 while distal end 104 is safely disposed within body base 54 to advantageously prevent hazardous exposure to needle cannula 4.

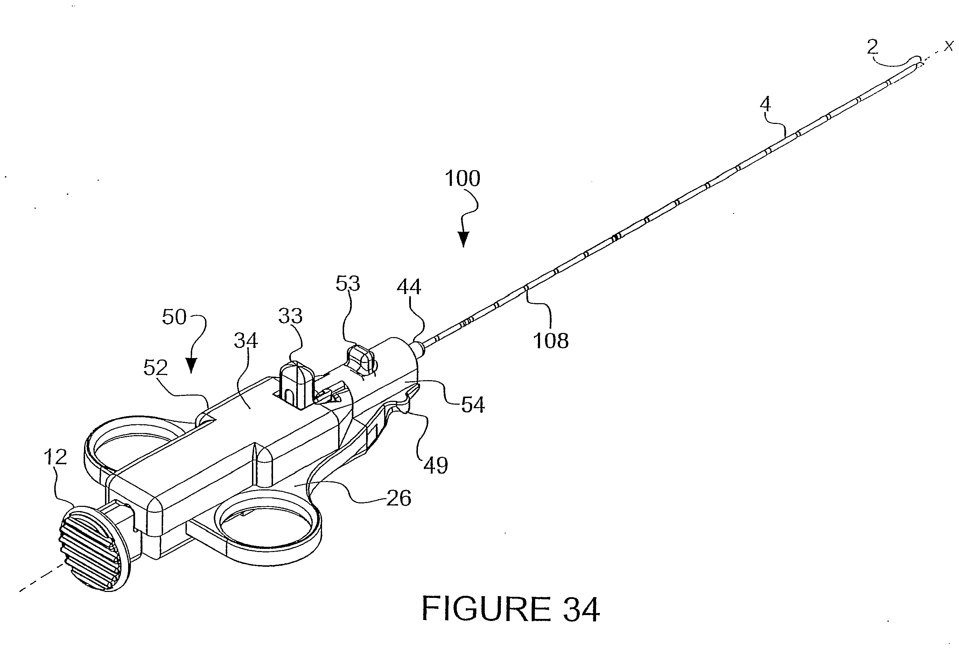

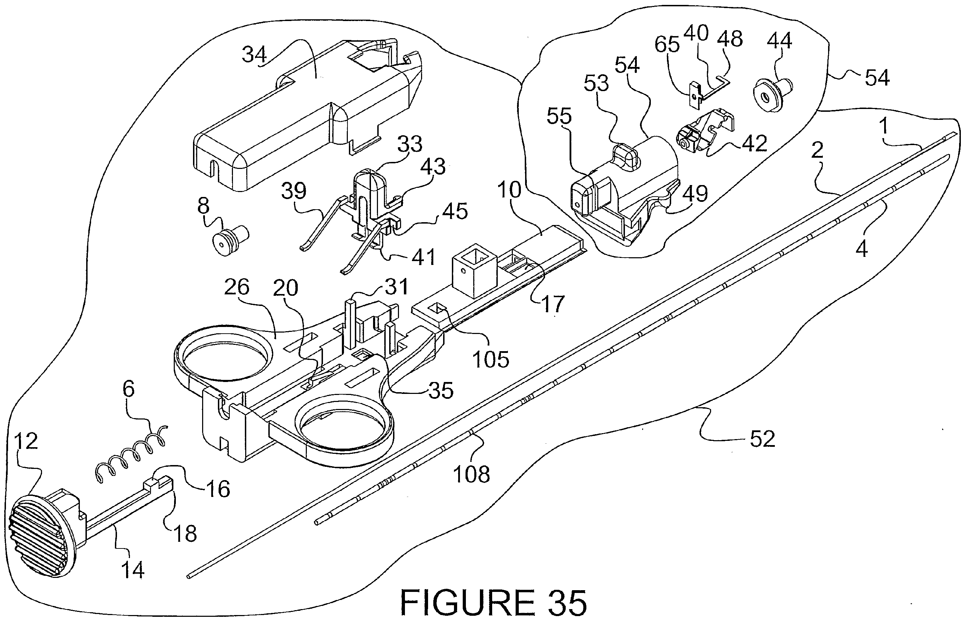

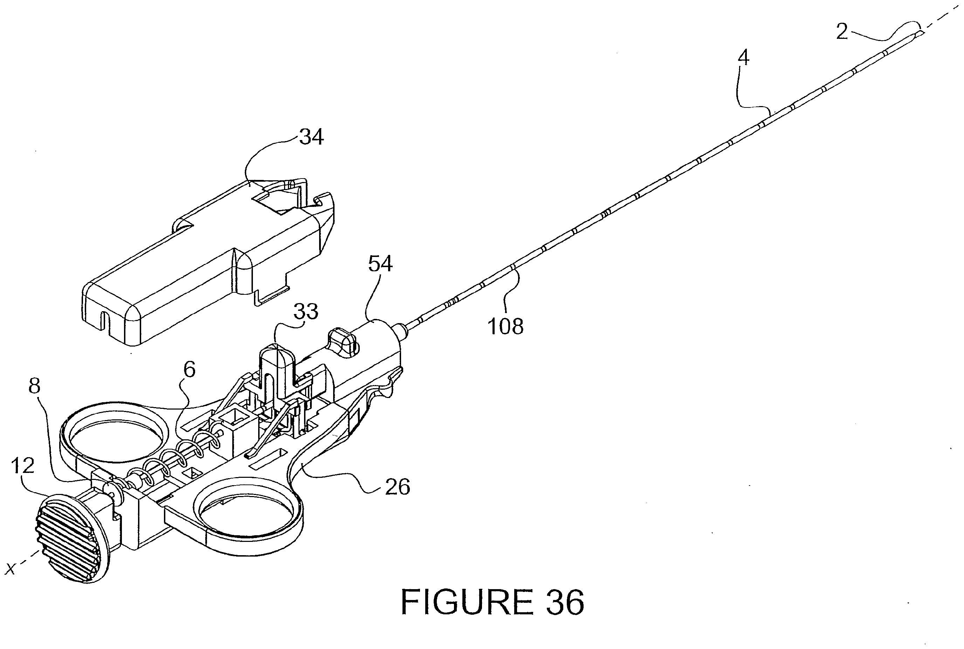

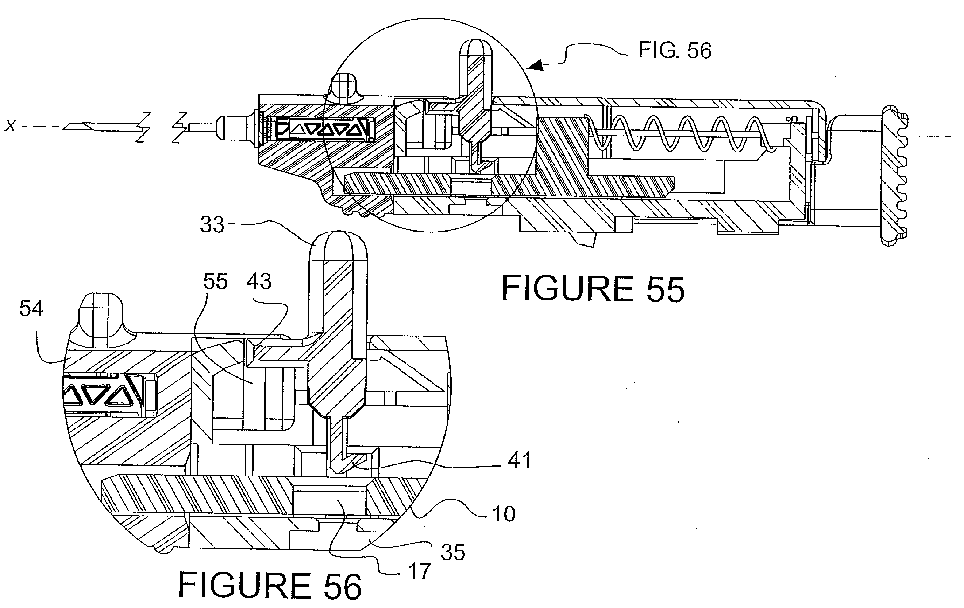

In an alternate embodiment, as shown in FIGURES 34-62, medical needle shield apparatus 100 has a body 50 that includes a first housing, such as, for example, body core 52. Body core 52 includes base handle 26. Body core 52 also includes an actuating mechanism that actuates a needle cannula 4 disposed therewith. The actuating mechanism, as previously described, advances needle cannula 4 to facilitate tissue sampling. An inner needle, such as, for example, stylette 2 is disposed for slidable movement within needle cannula 4. Needle cannula 4 is concentric with stylette 2. Stylette 2 has a recess 1 configured to capture tissue samples, as previously discussed.

Stylette 2 is fixed to the trigger-setting button 12, while being held concentric to the stylette 2 via bushing 8. The interaction between the trigger-setting button 12 and coring cannula slide 10 is accomplished by spring 6 and base handle 26. The spring 6 resides concentric to stylette 2, saddled over bushing 8, and against the proximal side of the coring cannula slide 10. The trigger-setting button 12 is supported by the base handle 26, so as to allow only axial motion of the stylette 2 within needle cannula 4. Trigger-setting arm 14 includes a trigger-setting tooth 16, which is configured for disposal within a groove 105 of coring cannula slide 10.

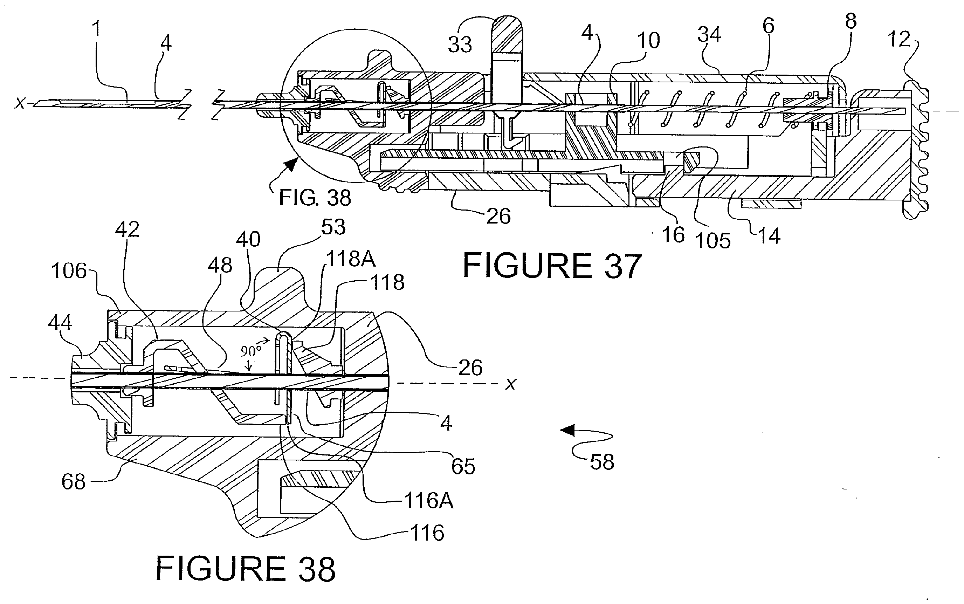

In a primary state of medical needle shield apparatus 100, as shown in FIGURES 34, 36 and 37, spring 6 is expanded and trigger-setting button 12 and coring cannula slide 10 are in a distal position on base handle 26. Needle cannula 4 and stylette 2 are attached to coring cannula slide 10 and trigger-setting button 12, respectively, each in a distal position. In such a distal position, recess 1 of stylette 2 is enclosed by needle cannula 4, as shown in FIGURE 37. Binding member 40 is in a non-binding or sliding orientation, as will be discussed, which allows needle cannula 4 to slide freely.

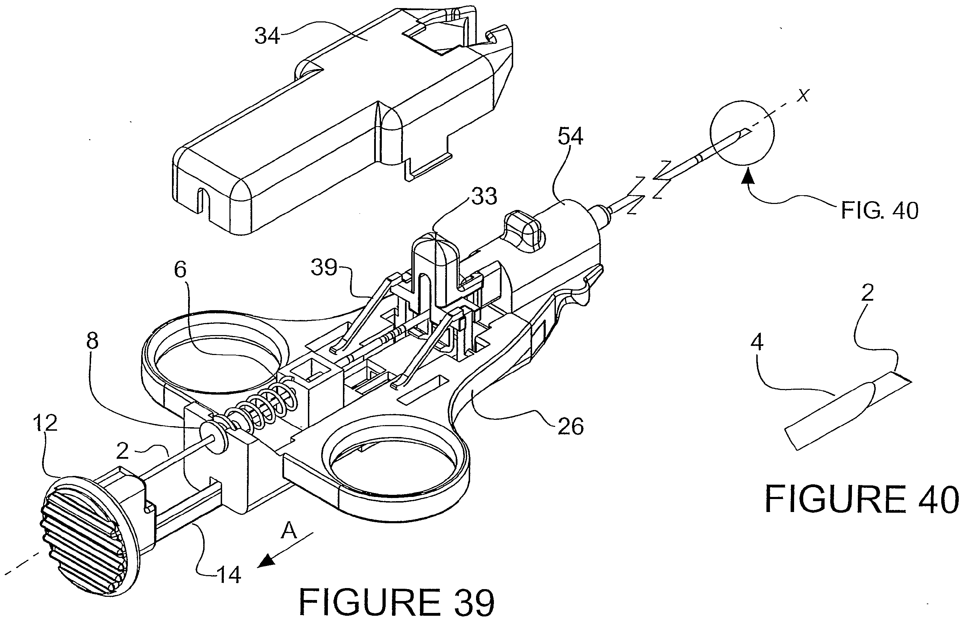

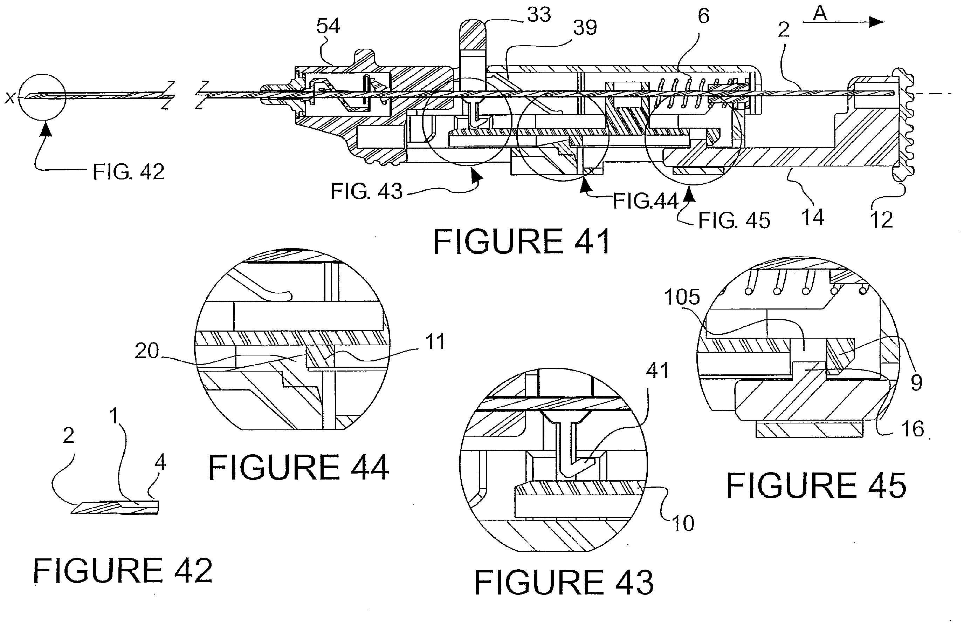

To set the actuating mechanism, trigger-setting button 12 is manipulated in a proximal direction along longitudinal axis x, in the direction of arrow A shown in FIGURES 39 and 41. Needle cannula 4 maintains enclosure of recess 1 of stylette 2, as shown in FIGURE 40. Trigger-setting arm 14 includes a trigger-setting tooth 16, which is configured for disposal within a groove 105 of coring cannula slide 10. Disposal of trigger-setting tooth 16 within groove 105 causes corresponding engagement with proximal slide stop 9, as shown in FIGURE 45. Correspondingly, a distal slide stop 11 of coring cannula slide 10 engages a compression lock 20, as shown in FIGURE 44. Proximal slide stop 9 and distal slide stop 11 thereby facilitate maintenance of the actuating mechanism in a set position. Proximal movement of trigger-setting button 12 also compresses spring 6 into a set position. In the set position, needle cannula 4 maintains enclosure of recess 1 of stylette 2, as shown in FIGURE 42. A button 33 and button locking tooth 41 are held above the slide action by a button spring return 39, which allows the coring cannula slide 10 to slide without interference.

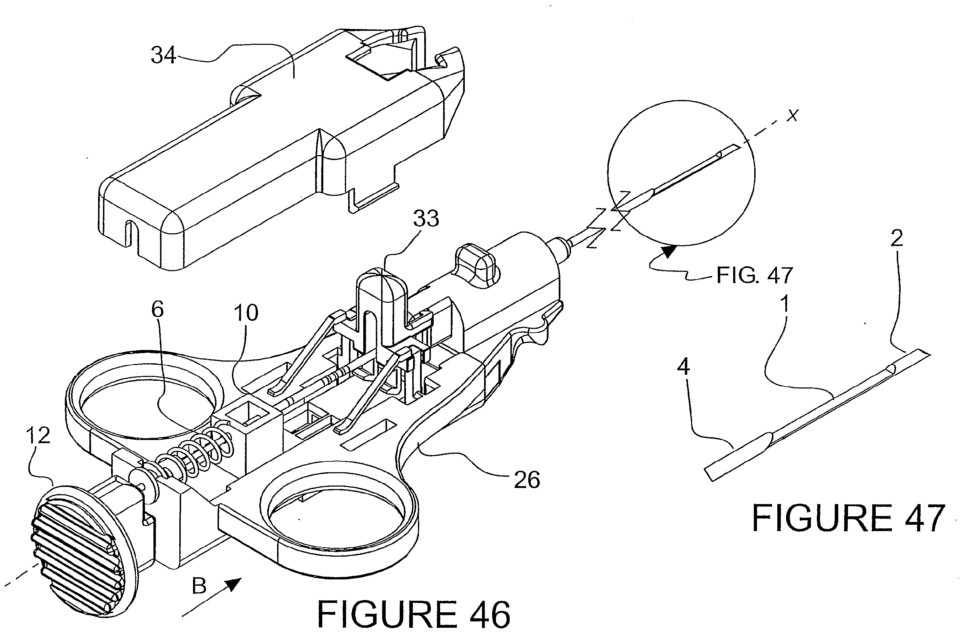

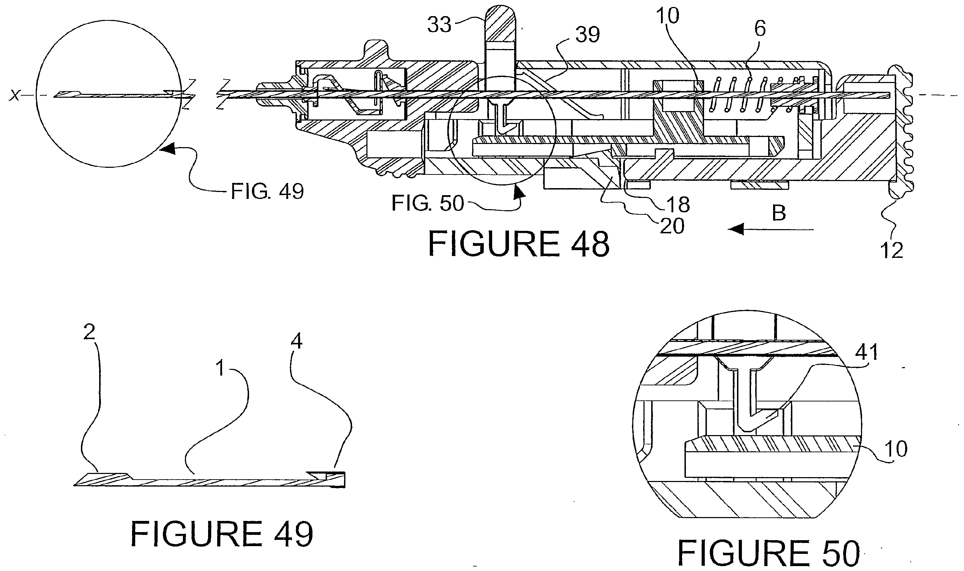

In contemplation of actuation of needle cannula 4, trigger-setting button 12 is manipulated in a distal direction, as shown by arrow B in FIGURES 46 and 48, until resistance is encountered. For example, a source of resistance to the distal manipulation can include engagement of a distal slide stop 11 with compression lock 20, as shown in FIGURE 44. The distal manipulation of trigger-setting button 12 forces stylette 1 forward and exposes recess 1, as shown in FIGURES 47 and 49, while spring 6 is maintained in compression. Button spring return 39 maintains button 33 and button locking tooth 41 above the slide action, as shown in FIGURE 50.

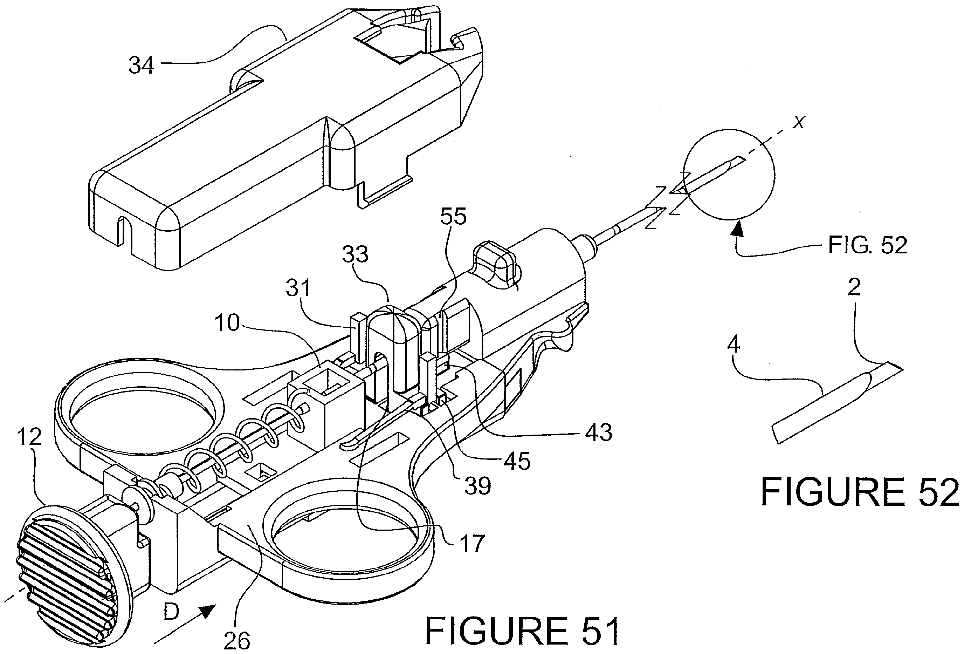

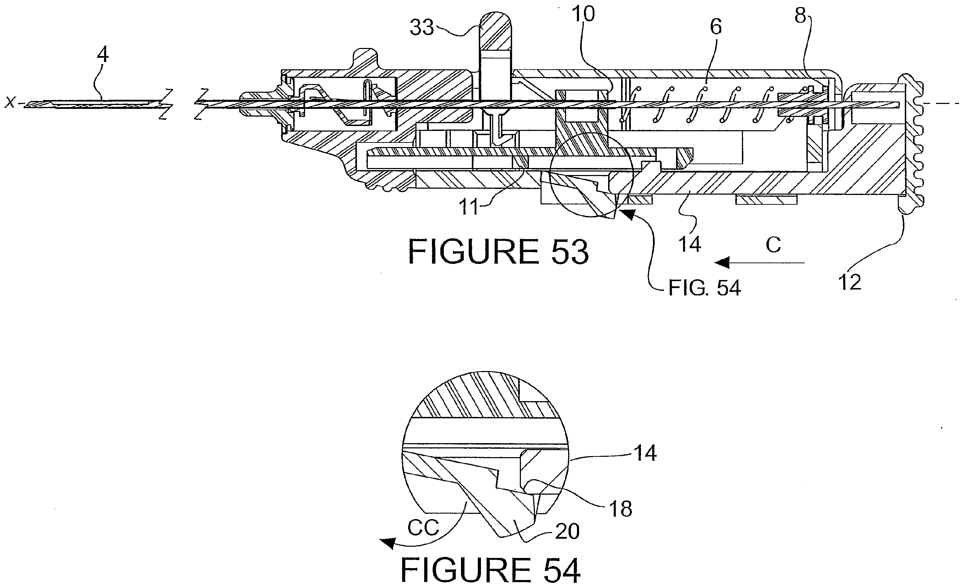

In an exemplary actuation, needle cannula 4 and stylette 2 are placed through soft tissue of a subject (not shown) to a desired location, which may be viewed via echogenic and depth markings 108. At the desired location, a tissue sample for biopsy enters recess 1. Trigger-setting button 12 is manipulated distally beyond the initial resistance of trigger core activating ramp 18 with compression lock 20, as shown by arrow C in FIGURE 53. Further manipulation of button 12 drives trigger core activating ramp 18 to deflect compression lock 20 such that distal slide stop 11 disengages from compression lock 20, as shown by arrow CC in FIGURE 54. Thus, coring cannula slide 10 is thrust, via spring 6, to a distal position, as shown by arrow D in FIGURE 51. Correspondingly, needle cannula 4 is thrust distally to core and enclose the soft tissue within recess 1, as shown in FIGURES 52 and 53. The captured soft tissue sample is then withdrawn from the subject. The captured soft tissue sample is removed by resetting the actuating mechanism and distally manipulating button 12 to expose recess 1, as described above, outside of the subject. Additional soft tissue sampling may be repeated with medical needle shield apparatus 100.

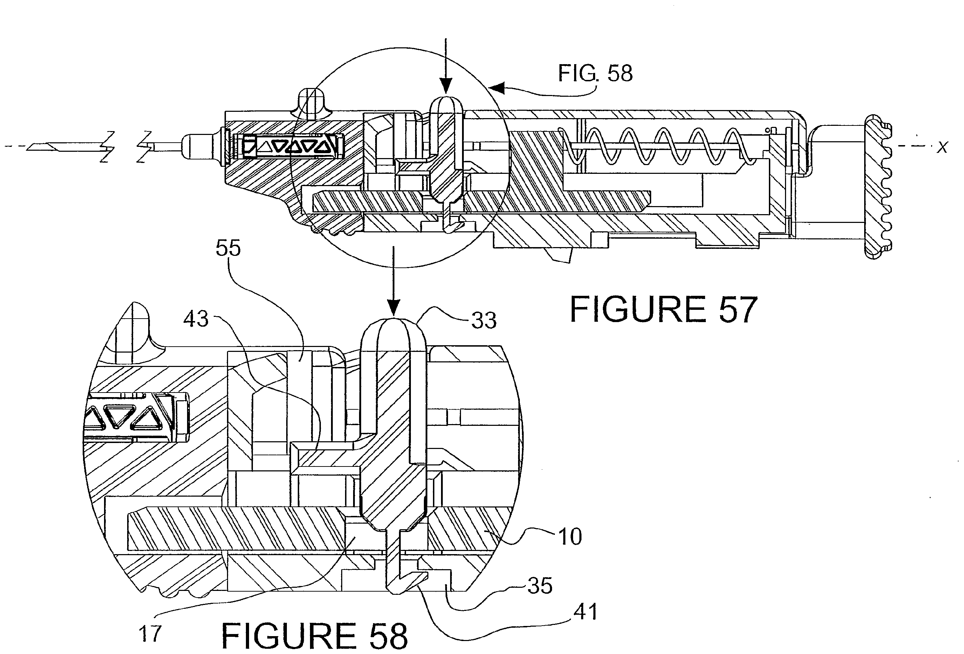

Body base 54 remains adjacent to body core 52 by means of a pre-activation locking channel 55, while attached to the pre-activation locking arm 43 of button 33. After soft tissue core samples are harvested, the medical needle shield apparatus 100 may readied for activation by fully depressing button 33 until it is flush with base cover 34. Button 33 may then slide vertically along button guide channel 45 interface with base guide rails 31, as shown in FIGURE 51. This action locks out coring cannula slide 10. Button locking tooth 41 slides through the slide lockout gap 17 and base lockout gap 35. It is contemplated that slide lockout gap 17 may be located at various positions along coring cannula slide 10. Button locking tooth 41 then engages the edge of base handle 26, which disables the pre-setting of and triggering of coring cannula slide 10, as shown in FIGURES 57 and 58. It is contemplated that the orientation of button locking tooth 41 may be varied.

Button 33 can only be depressed when the medical needle shield apparatus 100 is in its primary state. Slide lockout gap 17 assists as a physical verification when in line with button locking tooth 41. Consequently, button 33 may only be depressed when coring cannula slide 10 is in its primary state. If button 33 is depressed when the medical needle shield apparatus 100 is in any other state, button locking tooth 41 contacts the flat surface of coring cannula slide 10 and will not lock out.

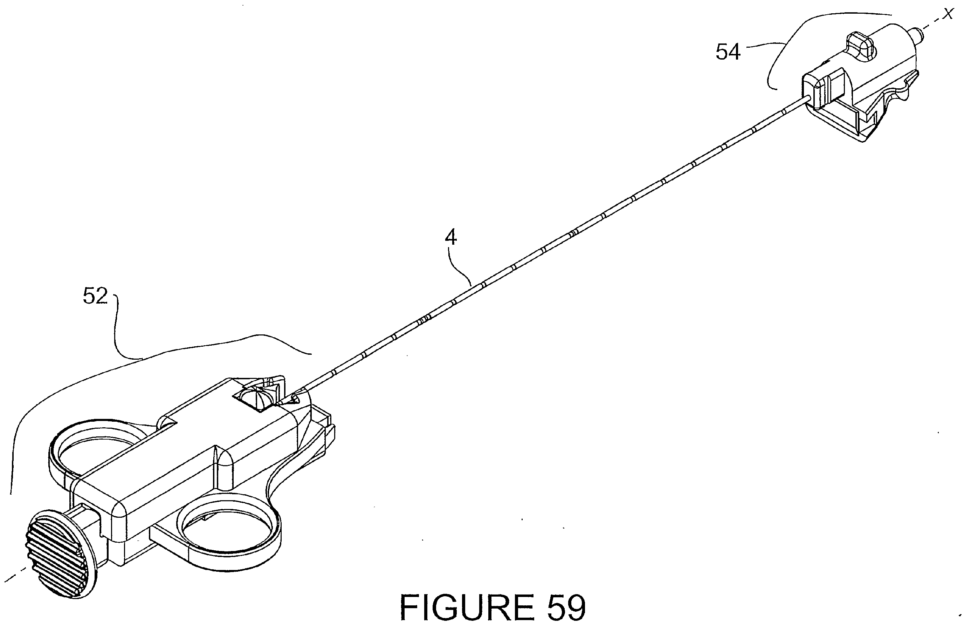

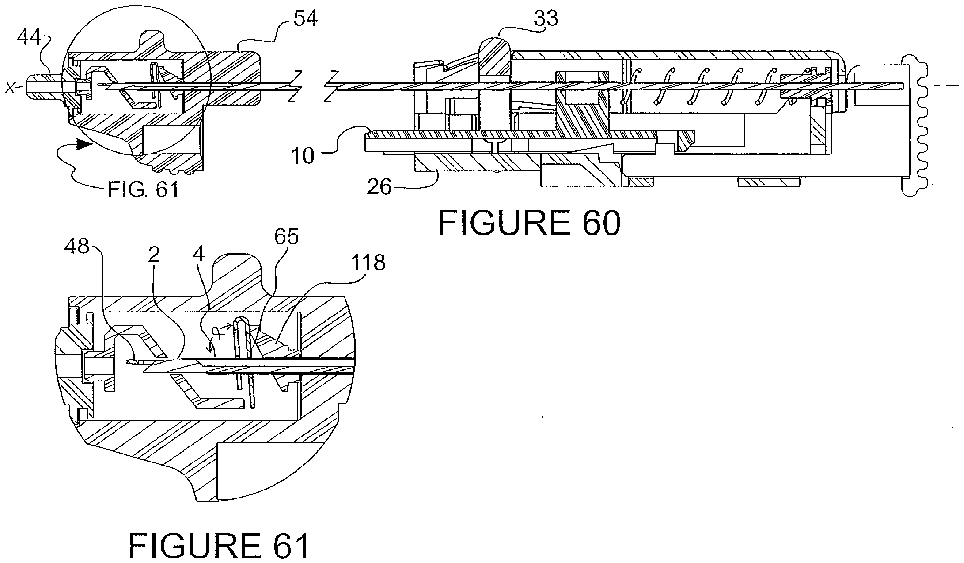

As the coring cannula slide 10 is locked out, base 54 is disengaged from body core 52 as pre-activation locking arms 43 slide past pre-activation locking channel 55, as shown in FIGURES 57-59. Base 54 is then extensible from a retracted position (FIG. 34) to an extended position (FIG. 59) to enclose a sharp distal end 104 of needle cannula 4. Base 54 may be gripped by means of thumb grip 53 or side grips 49. In the extended position, a binding member 40 disposed in base 54 binds to the needle cannula 4 as end-sensing member 48 detects the distal end of stylette 2 (FIGS. 60-61), as previously described.

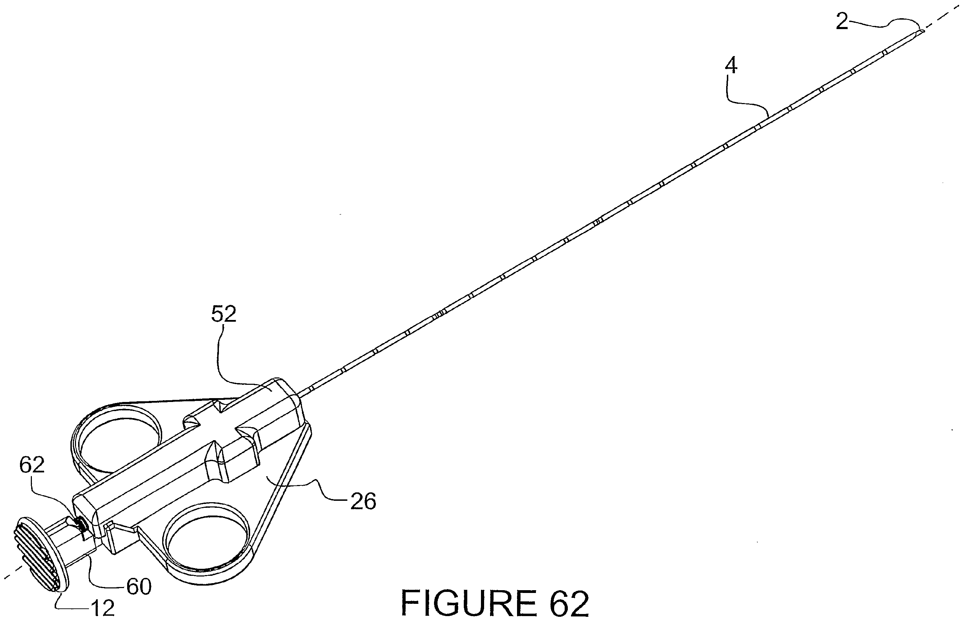

In an alternate embodiment, as shown in FIGURES 62-64, an energy storing component, such as spring 62, may be utilized to maintain the biopsy instrument in a pre-activated condition. In the pre-activated condition, the stylette 2 is covered by cannula 4 (FIG. 62). As trigger-setting arm 60 is set, spring 62 is in a compressed state and holds trigger-setting button 12 and trigger-setting arm 60 in a proximal position.

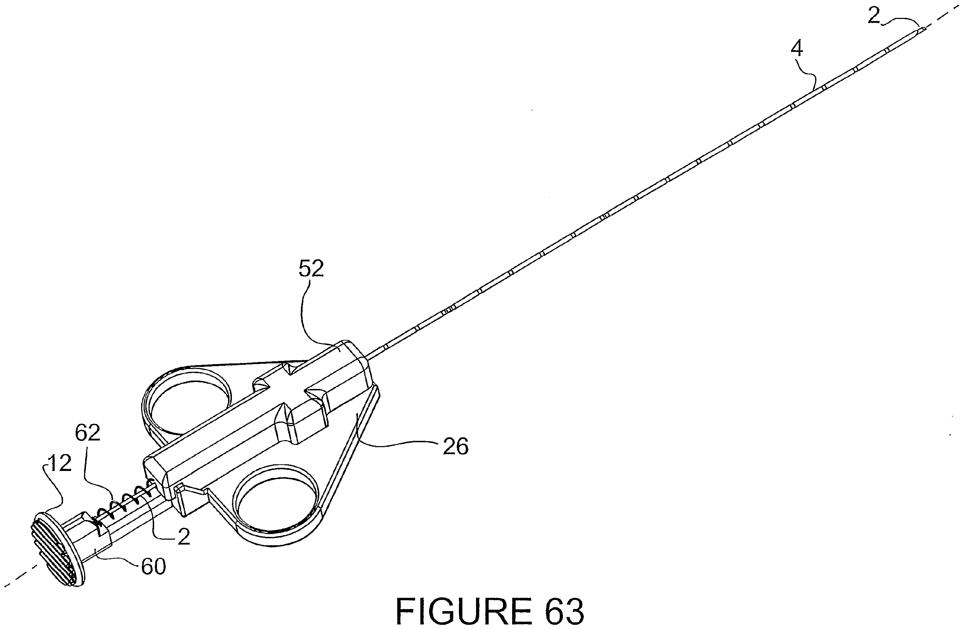

Energy storing component, such as spring 62, may be utilized to maintain the biopsy instrument in a pre-activated condition. In the pre-activated condition, the stylette 2 is covered by cannula 4 (FIG. 63). As trigger-setting arm 60 is set, spring 62 is in an extended state and holds trigger-setting button 12 and trigger-setting arm 60 in a proximal position. Without spring 62, stylette 2 is free to slide forward.

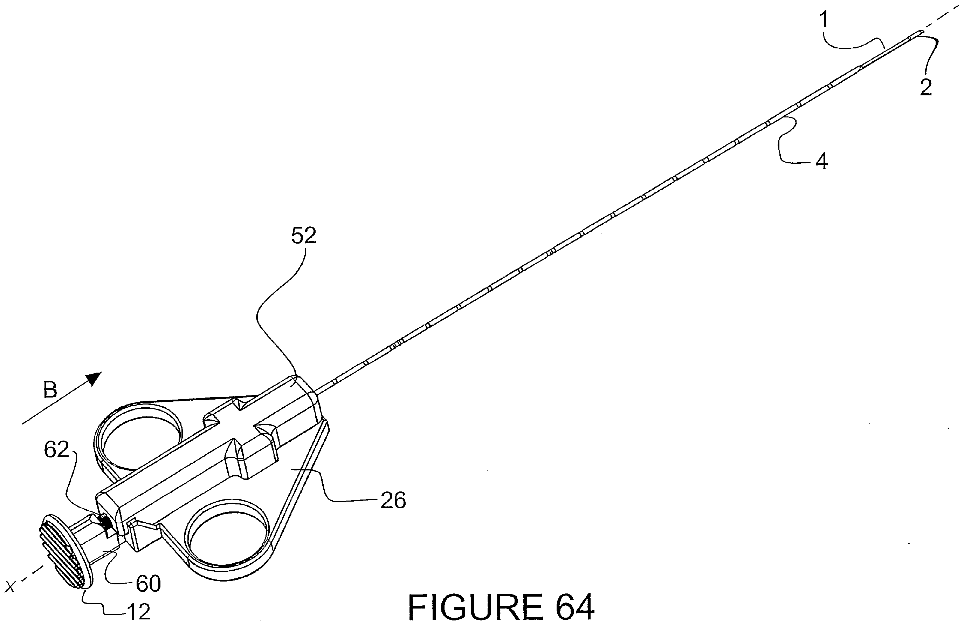

An external force in the direction of arrow B is required in the process of advancing stylette 2 to expose stylette recess 1 in a core harvesting function or in releasing a previously harvested core sample (FIG. 64). This external force must overcome the opposing force generated by spring 62 to advance stylette 2 in either function (FIG. 64). As stylette 2 requires repositioning within a specimen or as a previously harvested core sample has been disposed of, an operator then removes the external force applied to trigger-setting button 12. Stylette 2 then automatically withdraws inside needle cannula 4, thereby covering stylette recess 1 as shown in FIGURE 63. An operator is then able to harvest an additional core sample or to readjust within a tissue without manually drawing stylette 2 to its most proximal position. An added benefit is that spring 62 positions stylette 2 and/or trigger-setting arm 60 in a proximal position to lock out the gun portion of the instrument and prevent stylette 2 and/or trigger-setting arm 60 to from advancing once locked out.

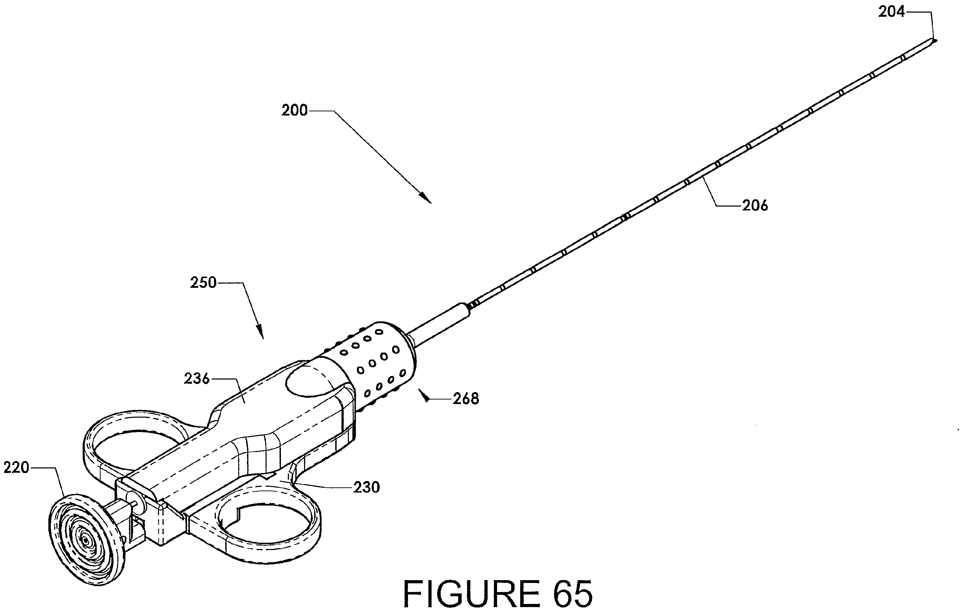

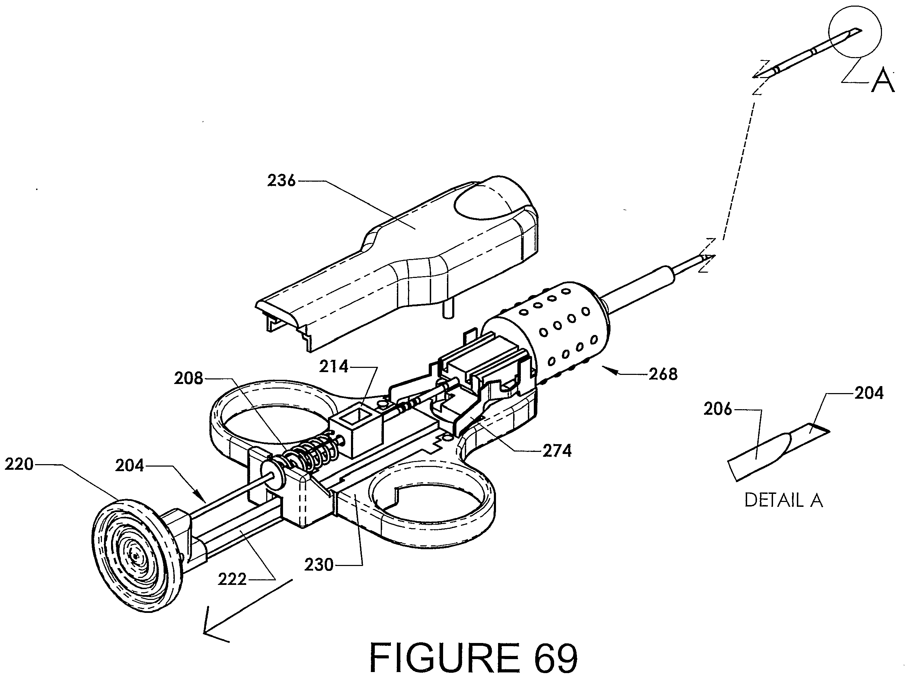

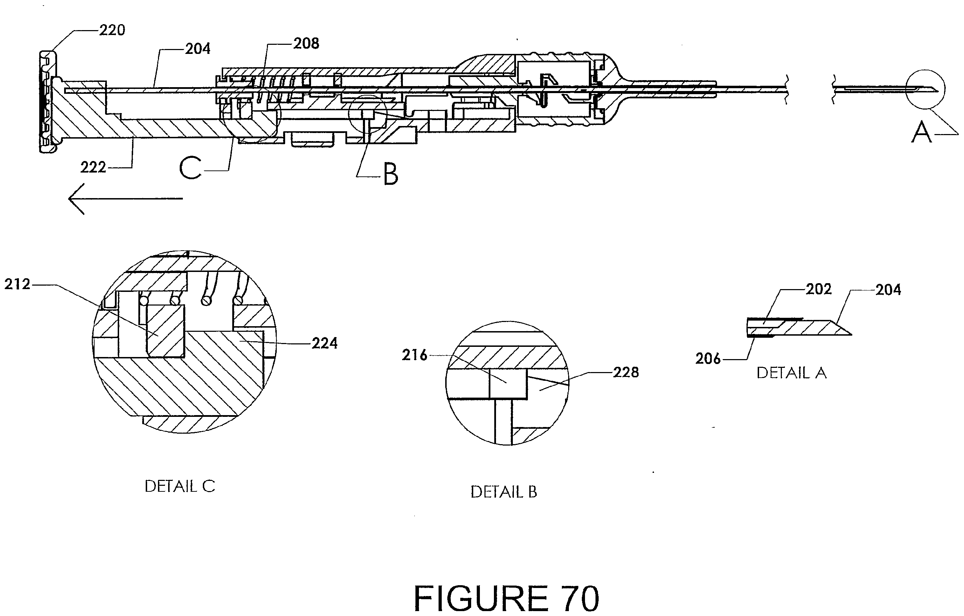

In an alternate embodiment, as shown in FIGURES 65-81, medical needle shield apparatus 200 has a body 250 that includes a first housing, such as, for example, base cover 236. Base cover 236 includes base handle 230. Base cover 236 also includes an actuating mechanism that actuates a needle cannula 206 disposed therewith. The actuating mechanism, as previously described, advances needle cannula 206 to facilitate tissue sampling. An inner needle, such as, for example, stylette 204 is disposed for slidable movement within needle cannula 206. Needle cannula 206 is concentric with stylette 204. Stylette 204 has a recess 202 configured to capture tissue samples, as previously discussed. Needle cannula 206 is sharpened, and may include echogenic features and depth markings.

In use, the medical needle shield apparatus 200 is first charged by pulling back on the trigger-setting button 220, which retracts the needle cannula 206 and stylette 204 and compresses the spring 208 that drives the needle cannula 206 when triggered later. The stylette 204 is not engaged by the spring 208. Second, the distal or sharp end of the device is inserted into the tissue to be sampled. This may be performed through a previously placed larger bore needle which is typically referred to as a "Coaxial Needle". Proper positioning of the distal end of the device in the tissue may be verified or guided by imaging means such as ultrasound. Third, the stylette 204 (which has remained retracted in the needle cannula 206 by virtue of friction and the resistance of penetrating the tissue) is advanced into the tissue to be sampled by manually urging the trigger-setting button 220 in the distal direction. Advancing the stylette 204 exposes the stylette recess 202. The tissue to be sampled then prolapses into the stylette recess 202. Fourth, upon full depression of the trigger-setting button 220, the compressed spring 208 and needle cannula 206 are released. The spring 208 drives the needle cannula 206 distally. The sharp tip of the needle cannula 206 cuts and captures the tissue which had previously prolapsed into the stylette recess 202. Fifth, the device is withdrawn from the patient and is once again charged as described in the first step above, while the stylette 204 is manually advanced exposing the sample which may now be transferred by some means into a container. Sixth, the second housing 268 is advanced past the base cover 236 which allows the locking arms 274 to release contact from the housing immobilizing surface 276 on the second housing 268. The locking arms 274 advance until a position is reached wherein the locking arms 274 are in the path of the coring cannula slide 214, thereby blocking distal advancement of the coring cannula slide 214. Seventh, the second housing 268 is advanced over the tip of the stylette 204. The second housing 268 continues to advance until it locks out over the tip and encases the stylette 204 tip within the second housing 268. Once the second housing 268 is removed from the body core 266, the coring cannula slide 214 will be blocked from distal advancement due to contact of the coring slide distal edge 280 with the locking arms 274. This final action renders the coring cannula slide 214 immovable in the distal direction.

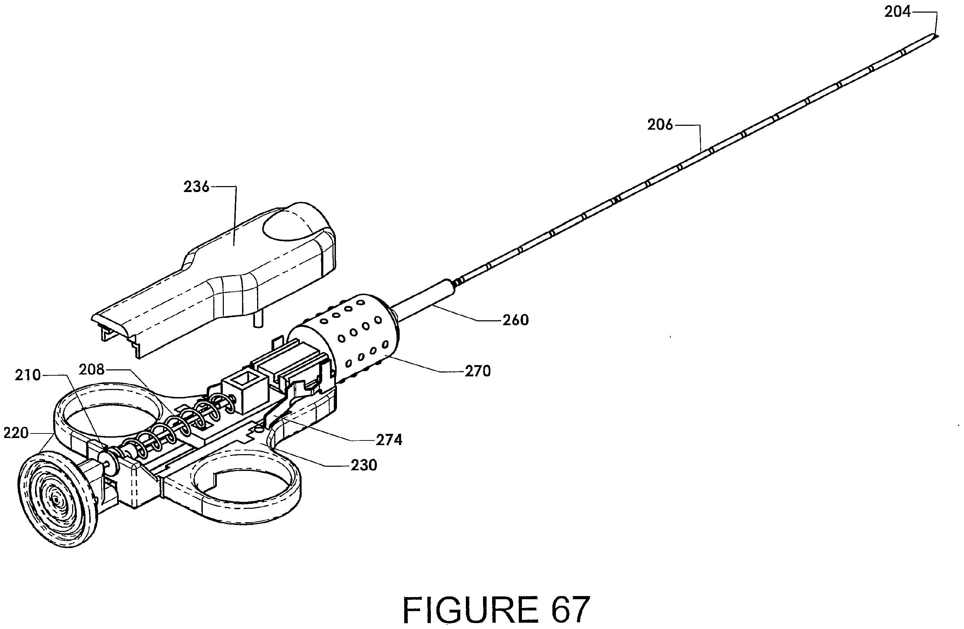

As shown in FIGURES 65-67, the needle cannula 206 is concentric with a stylette 204 which has a stylette recess 202 feature integral to the stylette 204. The needle cannula 206 is fixed to a coring cannula slide 214. The coring cannula slide 214 is encapsulated in a base handle 230 and covered by a base cover 236 (see FIGURES 65-68). The stylette 204 is fixed to the trigger-setting button 220, while being held concentric to the stylette 204 via the bushing 210 (see FIGURES 67-69).

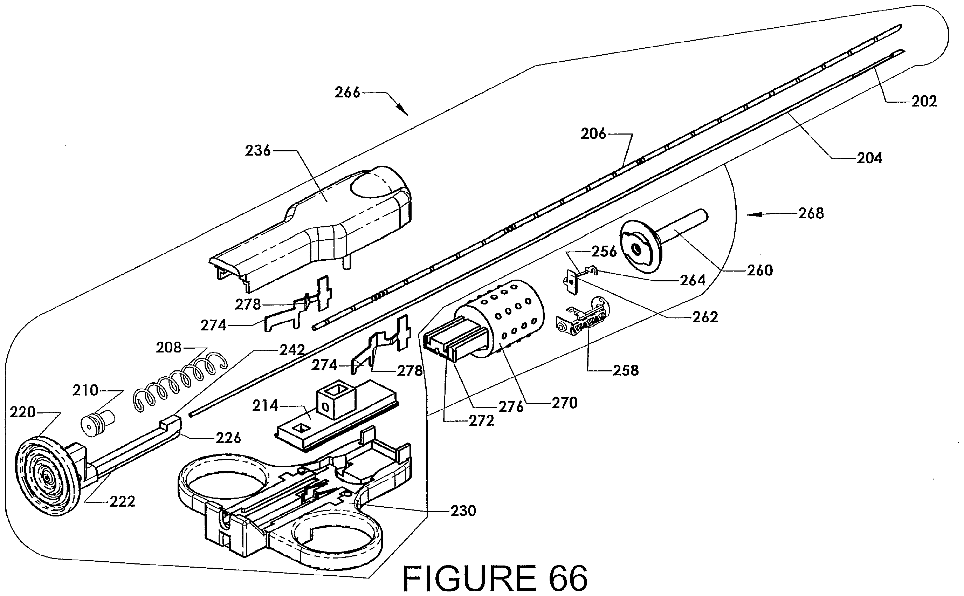

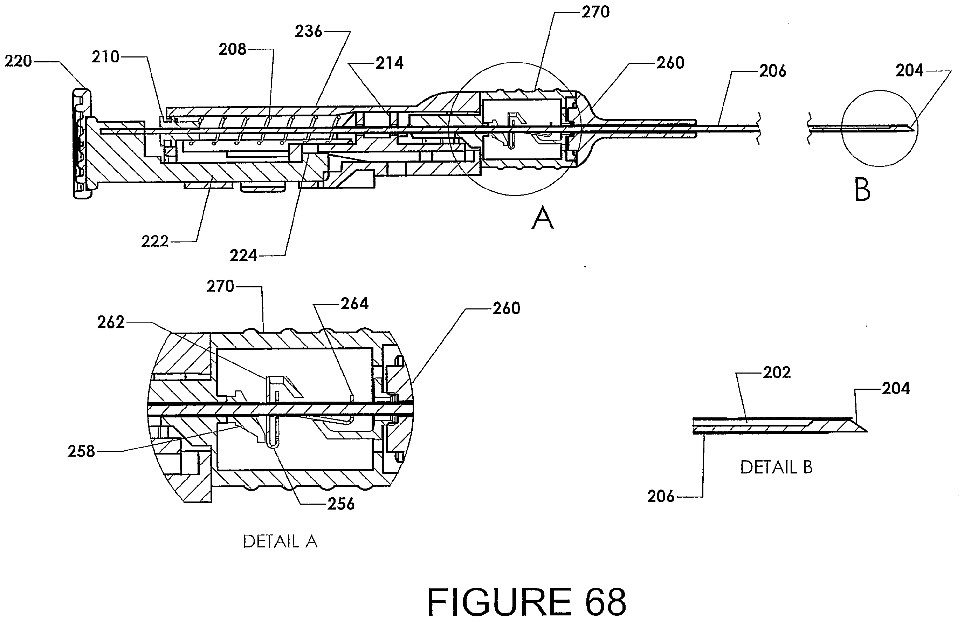

The interface of the trigger-setting button 220 and coring cannula slide 214 is accomplished by the spring 208 and base handle 230. The spring 208 resides concentric to the stylette 204, saddled over the bushing 210 and against the proximal side of the coring cannula slide 214. The trigger-setting button 220 is integral with the trigger-setting arm 222. This arm is supported by the base handle 230 in such a way as to only allow axial motion in conjunction with the stylette 204 within the needle cannula 206. Affixed to the trigger-setting arm 222 is the trigger-setting tooth 224. As shown in FIGURES 66 and 68, the trigger-setting tooth 224 resides within a groove of the coring cannula slide 214.

A second housing 268, such as, for example, a body base 270. Needle cannula 206 is disposed for slidable movement with body base 270 such that body base 270 is extensible from a retracted position to an extended position to enclose a sharp distal end of needle cannula 206.

The safety biopsy gun, as shown in FIGURES 65, 67, and 68, identify the primary state of the gun. The spring 208 is in full distal extension. The trigger-setting button 220 and coring cannula slide 214 are in their distal positions on the base handle 230 with their respective attached needles, stylette 204 and needle cannula 206, in their distal positions. The recess 202 is covered by the needle cannula 206 (see FIGURE 68). The binding member 256 is encapsulated within an inner housing 258, body base 270, and cap 260 (see FIGURES 67 and 68). The assembly of these components are defined as the second housing 268 (see FIGURE 66). While the aperture plate 262 of the binding member 256 is in a non-binding state, the needle cannula 206 slides freely along the axis within the aperture plate 262 as soft tissue core samples are harvested (see FIGURES 65-68).

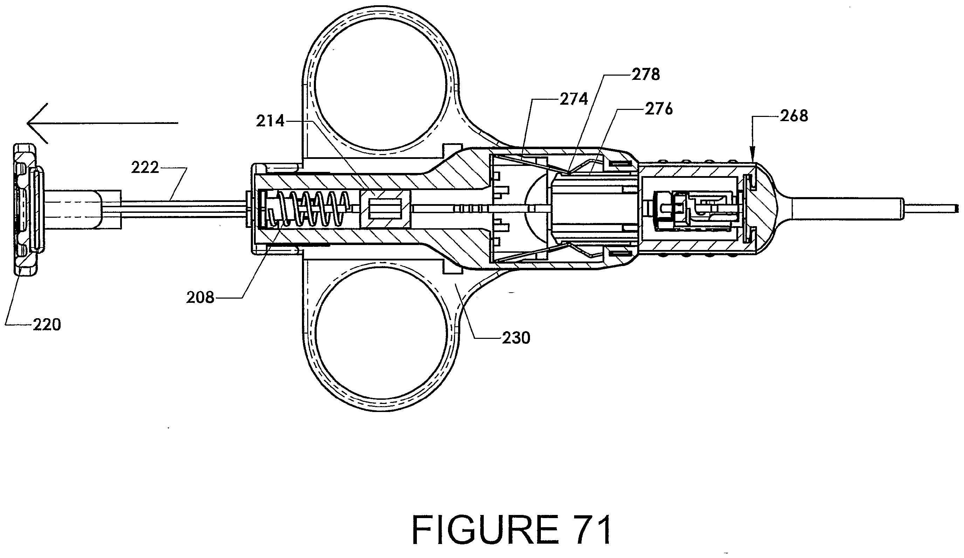

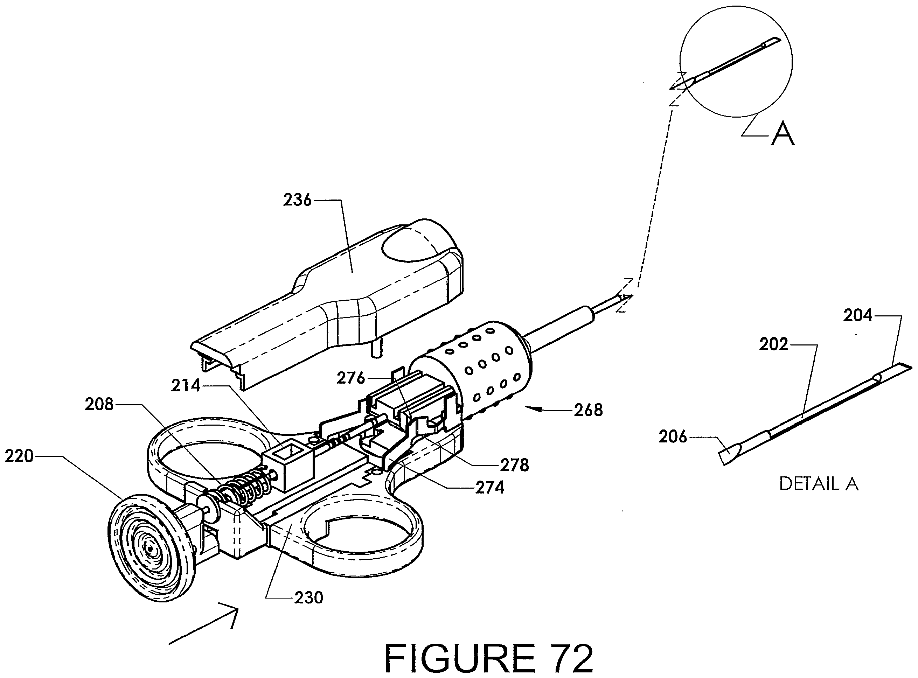

Trigger setting requires the trigger-setting button 220 to be pulled proximally along the axis of the trigger-setting arm 222 (see FIGURES 66, 69, and 70). This in turn engages the trigger-setting tooth 224 to the proximal slide stop 212. This engagement continues until the distal slide stop 216 engages the compression lock 228 (see FIGURE 72). This action also results in the spring 208 being in a compressed state. The recess 202 continues to be covered by the needle cannula 206 (see FIGURES 71 and 72). The needle cannula 206 continues to slide freely along the axis within binding member 256. The locking arms 274 are held away from the sliding action due to the interface of the arm immobilizing surface 278 and the housing immobilizing surface 276 on the second housing 268. This allows the coring cannula slide 214 to slide without interference (see FIGURE 71).

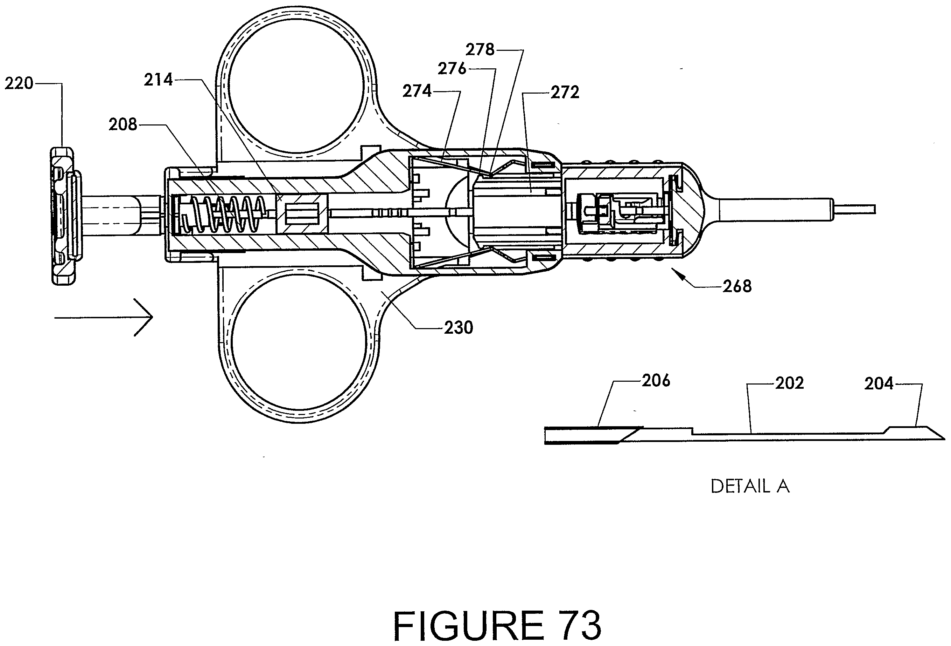

The trigger-setting button 220 is pushed distally until light resistance is met. The light resistance is felt when the trigger core activating ramp 226 comes in contact with the proximal edge of the compression lock 228 (see FIGURE 72). The coring cannula slide 214 continues to be in a compressed state with the spring 208. This exposes the recess 202 on the stylette 204 (see FIGURES 72 and 73). The locking arms 274 continue to be held away from the coring cannula slide 214 channel due to the interface of the arm immobilizing surface 278 and the housing immobilizing surface 276 on the second housing 268. The needle cannula 206 is then placed through soft tissue until a desired location is achieved. This location is viewed through the echogenic feature on the needle cannula 206.

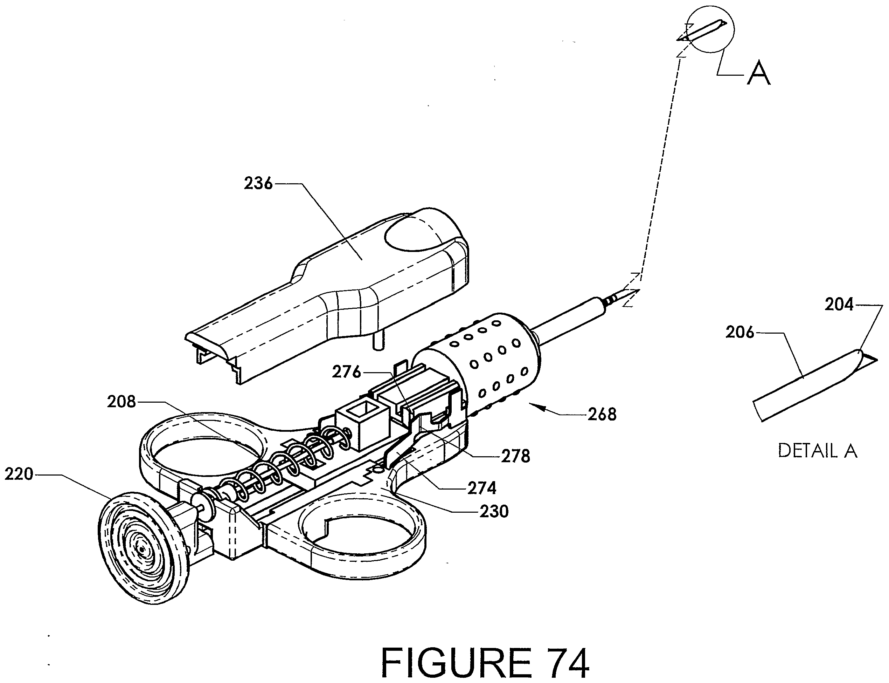

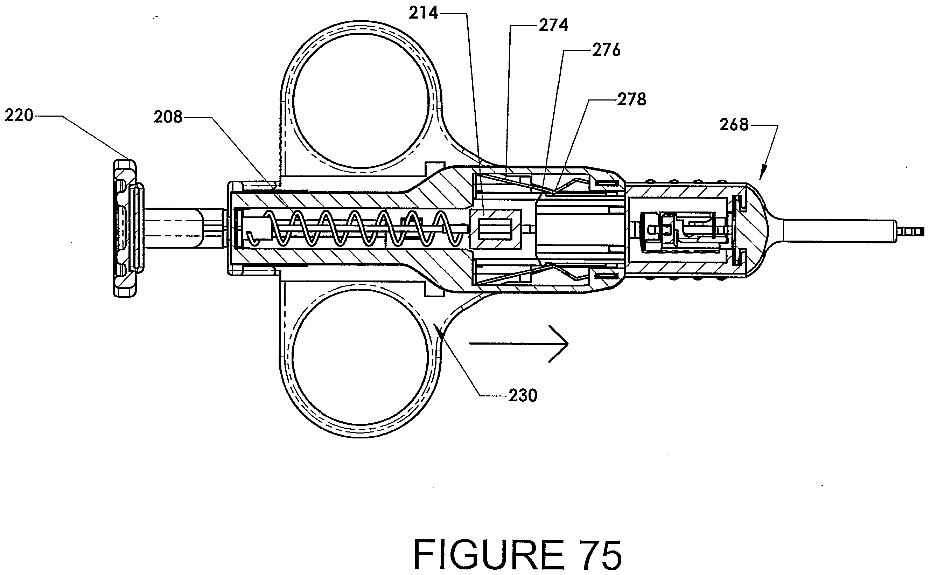

When the desired depth is achieved for harvesting a core sample, the trigger-setting button 220 is pressed distally beyond the initial contact/resistance of the trigger core activating ramp 226 against the proximal edge of the compression lock 228. This action deflects the compression lock 228 in such a way that the distal slide stop 216 disengages the compression lock 228 and thrusts the coring cannula slide 214 to its distal position via the compressed spring 208. This action allows the needle cannula 206 to move distally, thereby coring and encasing the soft tissue within the recess 202 (see Figure 74). This soft tissue core sample can then be withdrawn from the host. The locking arms 274 continue to be positioned away from the coring cannula slide 214 channel due to the interface of the arm immobilizing surface 278 and the housing immobilizing surface 276 on the second housing 268 (see FIGURE 75). The soft tissue core sample is then removed by repeating the steps described above outside of the host.

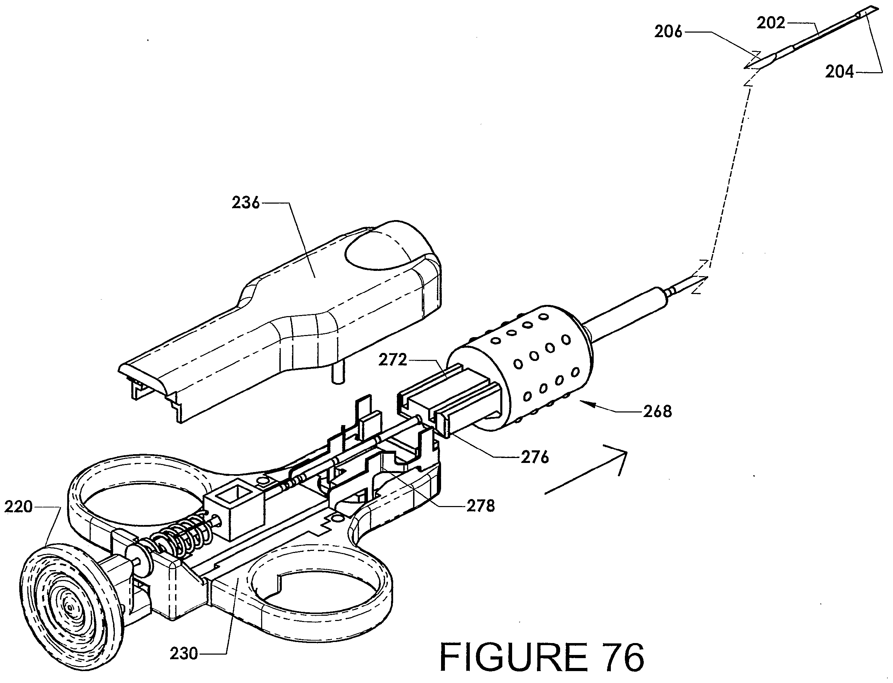

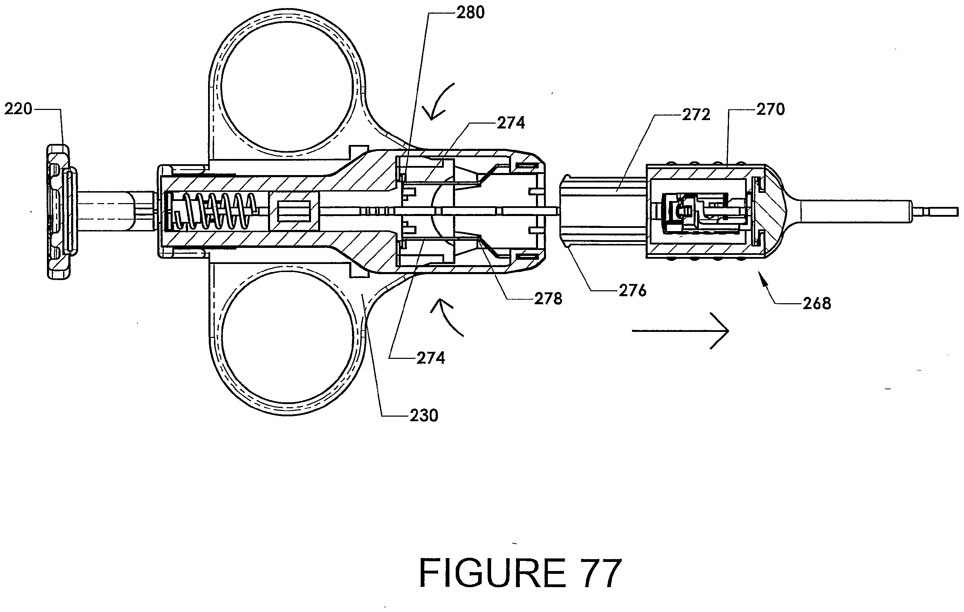

Once all soft tissue core samples are harvested, the second housing 268 may be advanced distally until it is disengaged from the body core 266 (see FIGURE 76). If the second housing 268 is advanced when the device is in the charged state, the locking arms 274, by means of the arm immobilizing contact surface 278, release contact from the housing immobilizing surface 276 on the body base 270. The locking arms 274 then advance into the path of the coring cannula slide 214, thereby blocking distal advancement of the coring cannula slide 214. The locking arms 274 disable the ability of the needle cannula 206 to advance and harvest additional samples and render the biopsy mechanism safe (see FIGURES 64 and 67). If the device is in the primary state (i.e., the stylette 204 is positioned distally, and not charged) when the second housing 268 is advanced, the locking arms 274, by means of the arm immobilizing contact surface 278, release contact from the housing immobilizing surface 276 on the body base 270. The Locking arms 274 advance inward until the locking arms 274 contact the sides of the coring cannula slide 214. If the device is then charged, the locking arms 274 then advance into the path of the coring cannula slide 214, thereby blocking distal advancement of the coring cannula slide 214. The locking arms 274 disable the ability of the needle cannula 206 to advance and harvest additional samples, which renders the biopsy mechanism safe (see FIGURES 76 and 77).

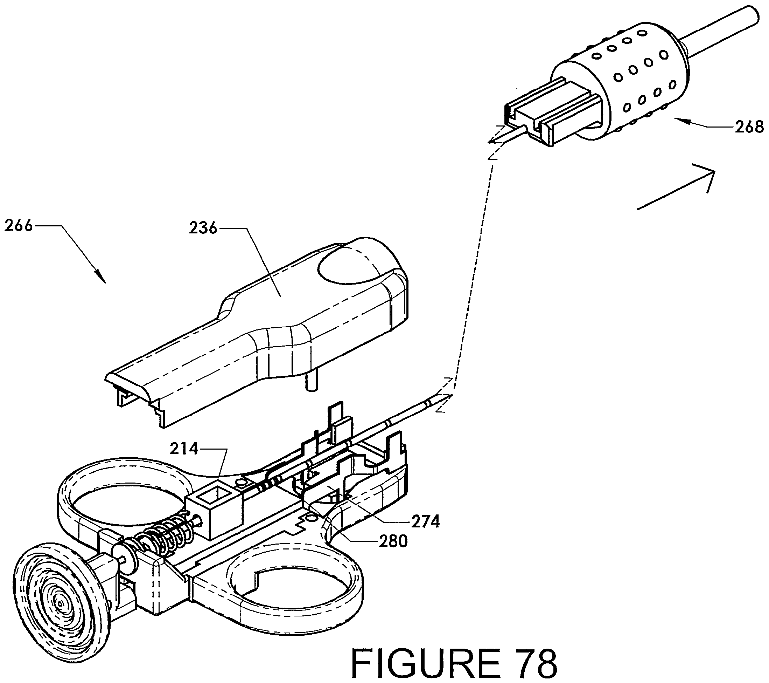

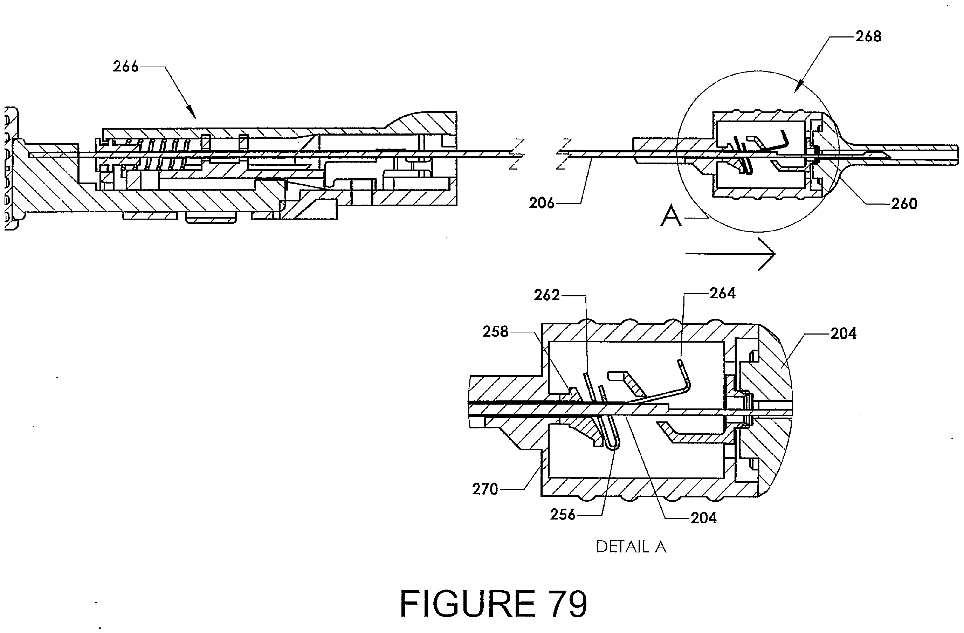

The second housing 268 continues to advance distally along the needle cannula 206 away from the body core 266. The second housing 268 continues to slide until the stylette 204 and needle cannula 206 lock out by the binding member 256 housed within the body base 270 (see FIGURES 78 and 79). The aperture plate 262 of the binding member 256 binds to the needle cannula 206 when the retainer 264 detects the end of the needle cannula 206 (see FIGURE 79).

The second housing 268 can be advanced and locked out over the needle cannula 206 and stylette 204 during any state of the device, when the needle cannula 206 is external to the soft tissue mass. However, the ability to disable distal advancement of the needle cannula 206 occurs when the second housing 268 is removed from the body core 266.

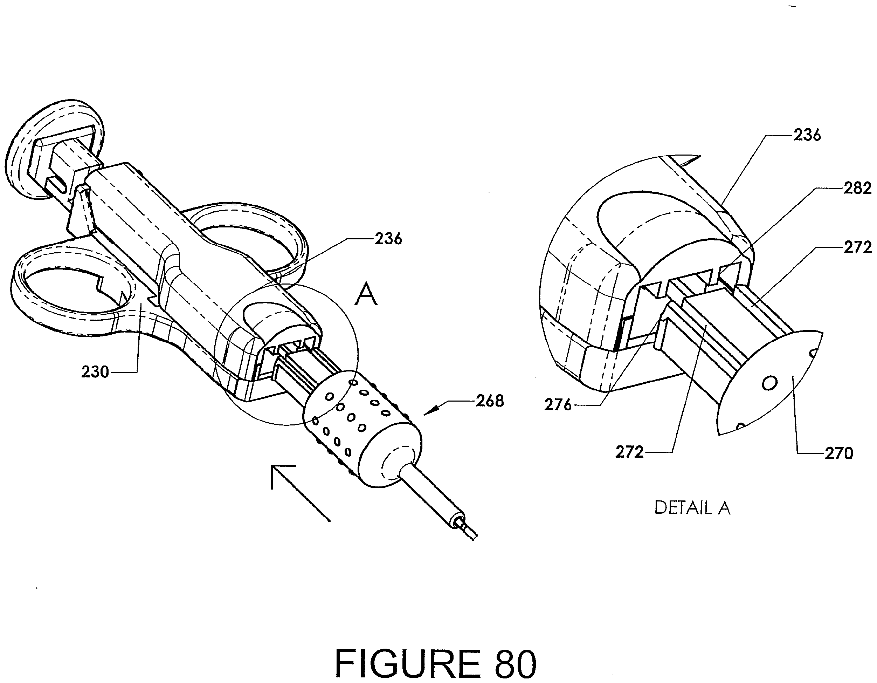

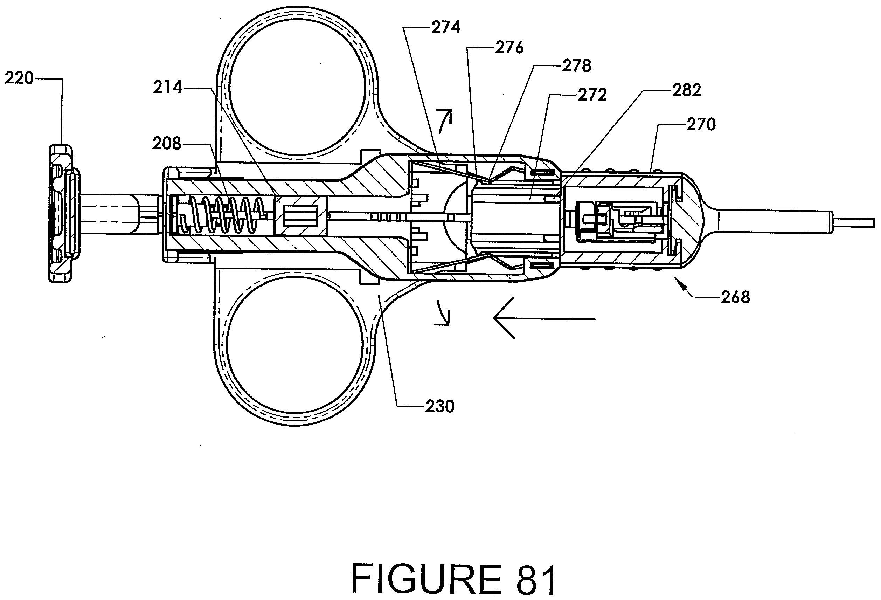

In the event that the second housing 268 is advanced distally past the body core 266 prematurely and not locked out over the end of the needle cannula 206, the second housing 268 may be reinserted into the body core 266 for additional tissue sampling. The safety keyway feature 272 must be aligned with the cover alignment keyway 282. The second housing 268 is advanced proximally until the locking arms 274 contact and are positioned away from the coring cannula slide 214 channel due to the interface of the arm immobilizing surface 278 and the housing immobilizing surface 276 on the second housing 268. Physical verification that the device has been reset is possible when the body base 270 is flush with the distal end of the base cover 236 (see FIGURES 80 and 81).

The invention of the present disclosure may be embodied in other specific forms without departing from the essential characteristics thereof. The present embodiments are therefore to be considered in all respects as illustrative and not restrictive, the scope of the invention being indicated by the appended claims rather than by the foregoing description, and all changes which come within the meaning of the claims are therefore intended to be embraced therein.

## Claims

1. A medical needle shield apparatus (100; 200) comprising:
a first housing (52; 236) and a second housing (54; 268), the first housing having a needle cannula (4; 206) disposed therewith, the needle cannula being disposed for slidable movement with the second housing such that the second housing is extensible from a retracted position to an extended position to enclose a distal end of the needle cannula;
a binding member (40; 256), disposed within the second housing and comprising binding surfaces (68), that define an aperture (66) configured for slidable receipt of the needle cannula between the retracted position and the extended position, a retainer (72) extending therefrom, such that the retainer is engageable with the needle cannula to prevent inclination of the binding member while the retainer is engaged with the needle cannula, and one or more drag inducing members (62) that engage the needle cannula during slidable receipt of the needle to create a drag force with the needle, the drag force and second housing facilitating inclination of the binding member relative to a longitudinal axis of the needle cannula once the retainer extends beyond the distal end of the needle such that the binding surfaces engage the needle cannula to prevent slidable movement of the needle cannula in the extended position of the second housing, **characterized in that** the first housing is configured to actuate the needle cannula, the needle cannula includes an inner needle disposed for slidable movement with the needle cannula and the first housing includes an actuating mechanism that actuates the needle cannula; and **in that** the second housing is releasably engageable with the first housing by means of an operable release (30,31, 32, 38, 114; 17, 33, 41, 43, 55; 274, 276, 278), such that activation of the operable release selectively locks movement between the needle cannula and the inner needle.

2. A medical needle shield apparatus as recited in claim 1, wherein the binding member includes a substantially planar aperture plate (65) that includes the binding surfaces that form the aperture.

3. A medical needle shield apparatus as recited in claim 2, wherein the at least one drag inducing member includes a pair of arms extending from the aperture plate.

4. A medical needle shield apparatus as recited in claim 1, wherein the second housing includes an inner housing (42; 258) that is disposed with the binding member.

5. A medical needle shield apparatus as recited in claim 4, wherein the inner housing defines at least one blocking member (116, 118) extending from an interior surface thereof, the at least one blocking member being engageable with the binding member for urging the binding member to a binding orientation.

6. A medical needle shield apparatus as recited in claim 1, wherein the inner needle includes a lateral recess (1; 202) disposed adjacent a distal end thereof.

7. A medical needle shield apparatus as recited in claim 1, further including a means for selectively locking movement between the needle cannula and inner needle.

8. A medical needle shield apparatus as recited in claim 1, wherein the inner needle includes a cutting edge.

9. A medical needle shield apparatus as recited in claim 1, wherein the needle cannula includes a cutting edge.

10. A medical needle shield apparatus as recited in claim 1, wherein the second housing includes a handle (26), the handle defining a cavity (102) configured for receipt of the first housing such that the first housing is releasably engageable with the second housing.

11. A medical needle shield apparatus as recited in claim 1, wherein the first housing includes a handle (26, 230; 236).

12. A medical needle shield apparatus as recited in claim 1, wherein the first housing includes a locking configuration (30) that mates with a groove (112) of the second housing to facilitate releasable engagement of the first housing and the second housing.

13. A medical needle shield apparatus as recited in claim 1, wherein the actuating mechanism includes a slide mounted (10; 214) with the needle cannula, the slide facilitating axial movement of the needle cannula.

14. A medical needle shield apparatus as recited in claim 13, wherein the actuating mechanism includes a biasing member (6; 62) that engages the slide to bias the needle cannula in a distal direction.

15. A medical needle shield apparatus as recited in claim 14, wherein the actuating mechanism includes a trigger (12; 20; 220) that is connected to the biasing member for actuation thereof.

16. A medical needle shield apparatus as recited in claim 14, including a spring means for maintaining the actuating mechanism in a proximal position.

## Patentansprüche

1. Medizinische Nadelschutzvorrichtung (100; 200), umfassend:
ein erstes Gehäuse (52; 236) und ein zweites Gehäuse (54; 268), wobei das erste Gehäuse eine damit angeordnete Nadelkanüle (4; 206) aufweist,
wobei die Nadelkanüle für eine gleitende Bewegung mit dem zweiten Gehäuse derart angeordnet ist, dass das zweite Gehäuse von einer eingefahrenen Position in eine ausgefahrene Position ausziehbar ist, um ein distales Ende der Nadelkanüle zu umschließen;
ein Bindungselement (40; 256), welches innerhalb des zweiten Gehäuses angeordnet ist und Bindungsoberflächen (68), welche eine Öffnung (66) definieren, die für gleitende Aufnahme der Nadelkanüle zwischen der eingefahrenen Position und der ausgefahrenen Position konfiguriert ist, einen sich davon erstreckender Halter (72), so dass der Halter mit der Nadelkanüle in Eingriff bringbar ist, um eine Neigung des Bindungselements zu verhindern, während der Halter mit der Nadelkanüle in Eingriff steht, und ein oder mehrere zuginduzierende Elemente (62), die mit der Nadelkanüle während der gleitenden Aufnahme der Nadel in Eingriff stehen, um eine Zugkraft mit der Nadel zu erzeugen, umfasst, wobei die Zugkraft und das zweite Gehäuse die Neigung des Bindungselements in Bezug auf eine Längsachse der Nadelkanüle ermöglichen, sobald sich der Halter über das distale Ende der Nadel hinaus erstreckt, so dass die Bindungsoberflächen in die Nadelkanüle eingreifen, um eine gleitende Bewegung der Nadelkanüle in der ausgefahrenen Position des zweiten Gehäuses zu verhindern, **dadurch gekennzeichnet, dass** das erste Gehäuse konfiguriert ist, um die Nadelkanüle in Bewegung zu versetzen, wobei die Nadelkanüle eine innere Nadel aufweist, die für eine gleitende Bewegung mit der Nadelkanüle angeordnet ist, und das erste Gehäuse einen In-Bewegung-Setz-Mechanismus aufweist, der die Nadelkanüle in Bewegung setzt; und dass das zweite Gehäuse mittels einer betätigbaren Entriegelung (30, 31, 32, 38, 114; 17, 33, 41, 43, 55; 274, 276, 278) lösbar mit dem ersten Gehäuse in Eingriff bringbar ist, so dass die Aktivierung der betätigbaren Entriegelung eine Bewegung zwischen der Nadelkanüle und der inneren Nadel selektiv blockiert.

2. Medizinische Nadelschutzvorrichtung nach Anspruch 1, wobei das Bindungselement eine im Wesentlichen planare Öffnungsplatte (65) aufweist, welche die Bindungsoberflächen aufweist, welche die Öffnung bilden.

3. Medizinische Nadelschutzvorrichtung nach Anspruch 2, wobei das mindestens eine zuginduzierende Element ein Paar von Armen aufweist, die sich von der Öffnungsplatte aus erstrecken.

4. Medizinische Nadelschutzvorrichtung nach Anspruch 1, wobei das zweite Gehäuse ein inneres Gehäuse (42; 258) aufweist, das mit dem Bindungselement angeordnet ist.

5. Medizinische Nadelschutzvorrichtung nach Anspruch 4, wobei das innere Gehäuse mindestens ein Blockierelement (116, 118) definiert, das sich von einer inneren Oberfläche desselben erstreckt, wobei das mindestens eine Blockierelement mit dem Bindungselement in Eingriff bringbar ist, um das Bindungselement zu einer Bindungsausrichtung zu drängen.

6. Medizinische Nadelschutzvorrichtung nach Anspruch 1, wobei die innere Nadel eine seitliche Aussparung (1; 202), die angrenzend an ein distales Ende davon angeordnet ist, aufweist.

7. Medizinische Nadelschutzvorrichtung nach Anspruch 1, ferner aufweisend ein Mittel zum selektiven Verriegeln der Bewegung zwischen der Nadelkanüle und der inneren Nadel.

8. Medizinische Nadelschutzvorrichtung nach Anspruch 1, wobei die innere Nadel eine Schneide aufweist.

9. Medizinische Nadelschutzvorrichtung nach Anspruch 1, wobei die Nadelkanüle eine Schneide aufweist.

10. Medizinische Nadelschutzvorrichtung nach Anspruch 1, wobei das zweite Gehäuse einen Griff (26) aufweist, wobei der Griff einen Hohlraum (102) definiert, der zur Aufnahme des ersten Gehäuses konfiguriert ist, so dass das erste Gehäuse lösbar mit dem zweiten Gehäuse in Eingriff bringbar ist.

11. Medizinische Nadelschutzvorrichtung nach Anspruch 1, wobei das erste Gehäuse einen Griff (26, 230; 236) aufweist.

12. Medizinische Nadelschutzvorrichtung nach Anspruch 1, wobei das erste Gehäuse eine Verriegelungsanordnung (30) aufweist, die mit einer Nut (112) des zweiten Gehäuses zusammenpasst, um ein lösbares Eingreifen des ersten Gehäuses und des zweiten Gehäuses zu ermöglichen.

13. Medizinische Nadelschutzvorrichtung nach Anspruch 1, wobei der Betätigungsmechanismus einen Schlitten (10; 214) aufweist, der mit der Nadelkanüle montiert ist, wobei der Schlitten die axiale Bewegung der Nadelkanüle unterstützt.

14. Medizinische Nadelschutzvorrichtung nach Anspruch 13, wobei der Betätigungsmechanismus ein Vorspannelement (6; 62) aufweist, das in den Schlitten eingreift, um die Nadelkanüle in einer distalen Richtung vorzuspannen.

15. Medizinische Nadelschutzvorrichtung nach Anspruch 14, wobei der Betätigungsmechanismus einen Auslöser (12; 20; 220) aufweist, der mit dem Vorspannelement zur Betätigung davon verbunden ist.

16. Medizinische Nadelschutzvorrichtung nach Anspruch 14, umfassend ein Federungsmittel zum Halten der Betätigungsmechanismus in einer proximalen Position.

## Revendications

1. Appareil de protection d'aiguille médicale (100 ; 200) comprenant :
un premier logement (52 ; 236) et un deuxième logement (54 ; 268), le premier logement ayant une canule d'aiguille (4 ; 206) disposée avec celui-ci, la canule d'aiguille étant disposée de manière à effectuer un mouvement coulissant avec le deuxième logement de sorte que le deuxième logement soit extensible d'une position rétractée vers une position déployée pour entourer une extrémité distale de la canule d'aiguille ;
un élément de liaison (40 ; 256) disposé dans le deuxième logement et comprenant des surfaces de liaison (68) qui définissent une ouverture (66) configurée pour recevoir en coulissement la canule d'aiguille entre la position rétractée et la position déployée, un dispositif de retenue (72) s'étendant à partir de celui-ci, de sorte que le dispositif de retenue puisse s'engager avec la canule d'aiguille pour empêcher l'inclinaison de l'élément de liaison pendant que le dispositif de retenue est engagé avec la canule d'aiguille, et un ou plusieurs élément(s) induisant une résistance (62) qui s'engagent avec la canule d'aiguille lors de la réception coulissante de l'aiguille pour créer une force de résistance avec l'aiguille, la force de résistance et le deuxième logement facilitant l'inclinaison de l'élément de liaison par rapport à un axe longitudinal de la canule d'aiguille une fois que le dispositif de retenue s'étend au-delà de l'extrémité distale de l'aiguille de sorte que les surfaces de liaison s'engagent avec la canule d'aiguille pour empêcher le mouvement coulissant de la canule d'aiguille dans la position déployée du deuxième logement, **caractérisé en ce que** le premier logement est configuré pour actionner la canule d'aiguille, la canule d'aiguille comporte une aiguille interne disposée de manière à effectuer un mouvement coulissant avec la canule d'aiguille et le premier logement comporte un mécanisme d'actionnement qui actionne la canule d'aiguille ; et **en ce que** le deuxième logement peut s'engager de manière amovible avec le premier logement au moyen d'un dispositif de libération fonctionnel (30, 31, 32, 38, 114 ; 17, 33, 41, 43, 55 ; 274, 276, 278), de sorte que l'activation du dispositif de libération fonctionnel bloque sélectivement le mouvement entre la canule d'aiguille et l'aiguille interne.

2. Appareil de protection d'aiguille médicale selon la revendication 1, dans lequel l'élément de liaison comporte une plaque à ouverture sensiblement plane (65) qui comporte les surfaces de liaison qui forment l'ouverture.

3. Appareil de protection d'aiguille médicale selon la revendication 2, dans lequel l'au moins un élément induisant une résistance comporte une paire de bras s'étendant depuis la plaque à ouverture.

4. Appareil de protection d'aiguille médicale selon la revendication 1, dans lequel le deuxième logement comporte un logement interne (42 ; 258) qui est disposé avec l'élément de liaison.

5. Appareil de protection d'aiguille médicale selon la revendication 4, dans lequel le logement interne définit au moins un élément de blocage (116, 118) s'étendant à partir d'une surface intérieure de celui-ci, l'au moins un élément de blocage pouvant s'engager avec l'élément de liaison pour pousser l'élément de liaison vers une orientation de liaison.

6. Appareil de protection d'aiguille médicale selon la revendication 1, dans lequel l'aiguille interne comporte un évidement latéral (1 ; 202) disposé de manière adjacente à une extrémité distale de celle-ci.

7. Appareil de protection d'aiguille médicale selon la revendication 1, comportant en outre un moyen pour bloquer sélectivement le mouvement entre la canule d'aiguille et l'aiguille interne.

8. Appareil de protection d'aiguille médicale selon la revendication 1, dans lequel l'aiguille interne comporte un bord tranchant.

9. Appareil de protection d'aiguille médicale selon la revendication 1, dans lequel la canule d'aiguille comporte un bord tranchant.

10. Appareil de protection d'aiguille médicale selon la revendication 1, dans lequel le deuxième logement comporte une poignée (26), la poignée définissant une cavité (102) configurée pour recevoir le premier logement de sorte que le premier logement puisse s'engager de manière amovible avec le deuxième logement.

11. Appareil de protection d'aiguille médicale selon la revendication 1, dans lequel le premier logement comporte une poignée (26, 230 ; 236).

12. Appareil de protection d'aiguille médicale selon la revendication 1, dans lequel le premier logement comporte une configuration de verrouillage (30) qui s'accouple avec une rainure (112) du deuxième logement pour faciliter l'engagement amovible du premier logement et du deuxième logement.

13. Appareil de protection d'aiguille médicale selon la revendication 1, dans lequel le mécanisme d'actionnement comporte un coulisseau (10 ; 214) monté avec la canule d'aiguille, le coulisseau facilitant le mouvement axial de la canule d'aiguille.

14. Appareil de protection d'aiguille médicale selon la revendication 13, dans lequel le mécanisme d'actionnement comporte un élément de sollicitation (6 ; 62) qui s'engage avec le coulisseau pour solliciter la canule d'aiguille dans une direction distale.

15. Appareil de protection d'aiguille médicale selon la revendication 14, dans lequel le mécanisme d'actionnement comporte un déclencheur (12 ; 20 ; 220) qui est relié à l'élément de sollicitation pour l'actionnement de celui-ci.

16. Appareil de protection d'aiguille médicale selon la revendication 14, comportant un moyen ressort pour maintenir le mécanisme d'actionnement dans une position proximale.
